# EUROPÄISCHE PATENTSCHRIFT

(11) **EP 2 011 887 B1**
(45) Veröffentlichungstag und Bekanntmachung des Hinweises auf die Patenterteilung: **25.02.2015**
(21) Anmeldenummer: 08166787.5
(22) Anmeldetag: 18.08.2005
(51) Int. Cl.: C12Q 1/68

(54) **Verfahren zur Unterscheidung zwischen nichtinfektiösen und infektiösen Ursachen eines Multiorganversagens**
Verfahren zur Unterscheidung zwischen nichtinfektiösen und infektiösen Ursachen eines Multiorganversagens
Beschichtungsverfahren für eine Form, Vorrichtung zur Durchführung dieses Verfahrens und Anwendung bei der Beschichtung von verlorenen Formen im Gießereibetrieb

(30) Priorität: 13.10.2004 DE 102004049897
(43) Veröffentlichungstag der Anmeldung: 07.01.2009
(62) Teilanmeldung aus: 05775921.9
(73) Patentinhaber: Analytik Jena AG, 07745 Jena (DE)
(72) Erfinder: Rußwurm, Stefan, 07743 Jena (DE); Reinhart, Konrad, 07743 Jena (DE)
(74) Vertreter: Winter, Brandl, Fürniss, Hübner, Röss, Kaiser, Polte - Partnerschaft mbB

(56) Entgegenhaltungen:
- PRUCHA M ET AL: "EXPRESSION PROFILING: TOWARD AN APPLICATION IN SEPSIS DIAGNOSTICS" SHOCK, Bd. 22, Nr. 1, Juli 2004 (2004-07), Seiten 29-33, XP008036997
- HELLER R A ET AL: "Discovery and analysis of inflammatory disease-related genes using cDNA microarrays" PROCEEDINGS OF THE NATIONAL ACADEMY OF SCIENCES OF USA, NATIONAL ACADEMY OF SCIENCE, WASHINGTON, DC.; US, Nr. 94, 1. Januar 1900 (1900-01-01), Seiten 2150-2155, XP002076789 ISSN: 0027-8424
- "Details for HG-U95AV2:2024_S_AT" INTERNET CITATION, [Online] 1. Oktober 2004 (2004-10-01), Seiten 1-3, XP007907827 Gefunden im Internet: URL:https://www.affymetrix.com/analysis/ne taffx/fullrecord.affx?pk=H> [gefunden am 2009-03-20]
- HASLINGER CHRISTIAN ET AL: "Microarray gene expression profiling of B-cell chronic lymphocytic leukemia subgroups defined by genomic aberrations and VH mutation status" JOURNAL OF CLINICAL ONCOLOGY, AMERICAN SOCIETY OF CLINICAL ONCOLOGY, US, Bd. 22, Nr. 19, 1. Oktober 2004 (2004-10-01), Seiten 3937-3949, XP009114229 ISSN: 0732-183X
- "Minutes of the 10th meeting of Working Party I, in Luxombour, 22.26 November 1971, BR/144/71, pages 63-66, items 117-120", , 1 January 1971 (1971-01-01), XP055081477, [retrieved on 2013-09-27]

## Beschreibung

Die vorliegende Erfindung betrifft die Verwendung von in vitro aus einer Patientenprobe erhaltenen Genexpressionsprofilen für die Unterscheidung zwischen nichtinfektiösen und infektiösen Ursachen eines Multiorganversagens gemäß Anspruch 1.

Weiterhin betrifft die vorliegende Erfindung neue Möglichkeiten der Unterscheidung zwischen nichtinfektiösen und infektiösen Ursachen eines Multiorganversagens von Patienten, die sich aus experimentell abgesicherten Erkenntnissen im Zusammenhang mit dem Auftreten von Änderungen der Genexpressionen (Transkription) bei Patienten mit Multiorganversagen ableiten lassen.

Trotz Fortschritten im pathophysiologischen Verständnis und der supportiven Behandlung stellt das Multiorgandysfunktionssyndrom (MODS) bzw. das Multiorganversagen (MOV) bei intensivmedizinischen Patienten die häufigste Todesursache dar und nimmt weltweit weiter zu. Die Folgen dieser Entwicklung sind nicht nur für den einzelnen Patienten erheblich, sondern haben enorme Auswirkungen auf die Kosten des Gesundheitswesens und den medizinischen Fortschritt in vielen Bereichen der Medizin.

Als Multiorganversagen bezeichnet man das gleichzeitig oder in rascher zeitlicher Abfolge auftretende Versagen von zwei oder mehr vitalen Organsystemen. Das Multiorgandysfunktionssyndrom (MODS) geht als initiale Organinsuffizienz dem MOV voraus [1]. Man spricht heute vom Multiorganversagen wenn zwei oder mehr Organe gleichzeitig oder nacheinander Funktionstörungen aufweisen, wobei ein chronisch persistierendes Organversagen auszuschließen ist [2]. Die Prognose des MOV hängt eng mit der Anzahl der beteiligten Organsysteme zusammen. Die Mortalität beträgt bei Versagen eines Organs innerhalb der ersten 24 Stunden 22%, nach 7 Tagen 41 %. Beim Versagen von drei Organsystemen steigt die Mortalität am ersten Tag auf 80 % und nach 4 Tagen auf 100 % an [3].

Zur klinischen Schweregradeinteilung des MODS und MOV wird regelmäßig der Multiple-Organ-Failure-Score (MOF-Score) von GORIS et al. [4] oder alternativ auch der Sepsis-related Organ Failure Assessment-(SOFA-) Score verwendet [5]. Der MOF-Score erlaubt eine schnelle und klinisch einfache Klassifikation der Organfunktion in drei Abstufungen. Ein MOF-Score > 4 wird in der klinischen Literatur regelmäßig als MOV bezeichnet [6]. Der SOFA-Score ist ein Punktesystem, welches die schnelle klinische Beurteilung der Funktion, jeweils in vier Schweregradstufen, folgende Organsysteme bewertet: Atmung (Lunge), Koagulation, Leber, Herz-Kreislaufsystem, zentrales Nervensystem und Niere.

Das MOV läuft klinisch in drei Phasen ab [7]:
1. Organ im Schock: Der auslösende pathophysiologische Mechanismus ist ein Perfusionsdefizit unterschiedlichster Genese. Dieses Geschehen spielt sich innerhalb von Stunden ab und hinterläßt noch keine bleibenden Schäden.
2. Organdysfunktion: Ein fortbestehendes Perfusionsdefizit innerhalb der nächsten Tage führt zur Entstehung eines SIRS (Systemic Inflammatory Response Syndrome, klassifiziert nach [8]) mit lokalen Ödemen und Zellschädigungen. Diese Phase wird als Multiorgandysfunktionssyndrom (MODS) bezeichnet.
3. Organversagen: Das fortbestehende Perfusionsdefizit führt zur Stase im Splanchnikusgebiet, wodurch es zur Superinfektion und Translokation von Endotoxinen aus dem Darm kommt. Das führt zu einer Potenzierung der klinischen Symptome und zum Vollbild der Sepsis. Aus der Organdysfunktion wird ein Organversagen.

MODS und MOV sind Krankheitsbilder mit einer komplizierten Pathophysiologie. Bis heute sind die genauen molekularen Ursachen der Entstehung und die Komplexizität der immunologisch-inflammatorischen Wirtsantwort auf schwere Infektion und Trauma, die zur Auslösung eines SIRS und zu den entsprechenden kardiozirkulatorischen Auswirkungen führen kann, nur unzureichend verstanden [9].

MODS und MOV können kann sowohl infektiologischer als auch nichtinfektiologischer Genese sein. MODS und MOV treten regelmässig als klinisch wichtige Komplikation bei Patienten mit Sepsis, nach traumatischen Schock, bei Patienten nach Operationen unter Einsatz der Herz-Lungen-Maschine, nach Organtransplantationen u.v.m. auf (Abbildung 1). Ein wichtiger Pathomechanismus für die Entstehung von MODS und MOV ist die Entwicklung eines systemischen Inflammationssyndromes (SIRS, [8]). Die im Rahmen eines SIRS initiierten pathophysiologischen Prozesse involvieren nicht nur alle Komponenten des Immunsystems, sondern beeinträchtigen das kardiozirkulatorische System in allen Ebenen und beschränken sich nicht nur auf Myokarddepression und Vasodilatation. Die kardiozirkulatorischen Veränderungen vor allem auf Ebene der Mikrozirkulation bilden die gemeinsame Endstrecke und resultieren in einer Gewebehypoxie, die als wichtiger Kofaktor in der Pathogenese des Multiorganversagens gilt.

Abbildung 1 beschreibt exemplarisch die aus heutiger Sicht wichtigsten Mechanismen der Entstehung von MODS und MOV [10]: Ein überaktives Immunsystem scheint bei der Entstehung des Multiorganversagens eine zentrale Rolle zu spielen. Dabei nimmt das Endothel durch Sekretion von Zytokinen und durch Vermittlung der Leukozyten-Adhäsion eine zentrale Schlüsselrolle ein. In den Endothelzellen werden Signaltransduktionskaskaden aktiviert, die letztendlich zur Expression und Aktivierung von Transkriptionsfaktoren führen.

Das noch lückenhafte Wissen über die Abläufe in der Frühphase des MODS und MOV ist der Grund dafür, das es bis heute keine sensitive/spezifische Diagnostik, die zwischen infektiösen und nichtinfektiösen Ursachen diskriminieren kann, existiert. Neuartige Biomarker und Diagnostika, nunmehr auch auf Genexpressionsebene, können die für die Früherkennung des Multiorganversagens sowie zur Unterscheidung zwischen infektiösen und nichtinfektiösen Ursachen eines MODS und MOV essentiellen diagnostischen Informationen liefern und stellen darüber hinaus einen wichtigen Beitrag zur Aufklärung der pathophysiologischen Mechanismen systemischer Inflammationen dar.

Die klinisch häufig benutzten Frühsymptome wie Fieber, Leukozytose, Tachykardie und Tachypnoe sind bei der Diagnose eines MODS oder MOV sowie bei der Unterscheidung zwischen infektiösen und nichtinfektiösen Ursachen eines MODS und MOV völlig unspezifisch. Parameter, welche die Mikrozirkulationsstörungen früh erfassen, wie pH-Veränderungen der Drammukosa [11] und Laktatspiegel im Kapillarbett [12,13], das Auftreten einer respiratorischen Insuffizienz, deren Ursache nicht in der Lunge liegt [2],der Anstieg der Leukozyten-Elastase [14,15], die Höhe des Neopterin-Spiegels [16], die Aktivierung polymorphkerniger Leukozyten und die Höhe des IL-6-Spiegels [17] sind als Frühparameter für die spätere Entstehung von MODS und MOV bedingt geeignet, können aber keinen Beitrag zur Unterscheidung zwischen infektiösen und nichtinfektiösen Ursachen eines MODS und MOV machen. Es besteht daher ein dringender Bedarf für neue diagnostische Verfahren, welche die Fähigkeit des Fachmanns verbessern sollen, nichtinfektiöses von infektiösen MODS oder MOV frühzeitig zu unterscheiden, und dem Ansprechen auf spezifische Behandlungen Aussagen abzuleiten.

Genau die Unterscheidung zwischen infektiösen und nichtinfektiösen Ursachen eines MODS und MOV ist aber von höchster medizinischer Bedeutung, da mittels einer solchen Unterscheidung z.B. Antibiotika spezifischer eingesetzt werden können, was neben einer Vermeidung von Nebenwirkungen durch den unspezifischen Einsatz von Antibiotika auch zu einer erheblichen Kosteneinsparung beiträgt. Gleichfalls können so den Patienten sehr belastende sowie Zeit- und Personal-intensive diagnostische Massnahmen (z.B. Transport zum CT/MRT) zur Identifizierung des jeweiligen Infektionsortes, die Durchführung umfangreicher mikrobiologischer Methoden (z.B. die Untersuchung von Blutkulturen, die ebenfalls mit der Abnahme großer Mengen an Blut verbunden sind) aber auch der risikoreiche Austausch aller mit dem Patienten verbundener Plastikmaterialien wie Venenkatheder etc. bei Vorliegen eines nichtinfektiösen MODS oder MOV vermieden werden. Vice versa kann die schnelle Identifikation infektiöser Ursachen eines MODS oder MOV die zeitnahe Einleitung solcher Maßnahmen und damit die Reduktion der Sterblichkeit sicherstellen.

Technologische Fortschritte, insbesondere die Entwicklung der Microarray-Technologie, versetzen den Fachmann nun in die Lage, 10000 oder mehr Gene und deren Genprodukte gleichzeitig zu vergleichen. Die Anwendung solcher Microarray-Technologien kann nun Hinweise auf den Status von Gesundheit, Regulationsmechanismen, biochemischer Wechselwirkungen und Signalübertragungsnetzwerken geben. Das Verbessern des Verständnisses darüber, wie ein Organismus auf Infektionen reagiert, sollte die Entwicklung von verstärkten Erkennungs-, Diagnose- und Behandlungsmodalitäten für Sepsis-Erkrankungen erleichtern.

Microarrays stammen vom "Southern blotting" [19] ab, was die erste Herangehensweise darstellt, DNA-Moleküle in einer räumlich ansprechbaren Art und Weise auf einer festen Matrix zu immobilisieren. Die ersten Microarrays bestanden aus DNA-Fragmenten, oft mit unbekannter Sequenz, und wurden auf eine poröse Membran (normalerweise Nylon) punktweise aufgebracht. Routinegemäß wurden cDNA, genomische DNA oder Plasmid-Bibliotheken verwendet, und das hybridisierte Material wurde mit einer radioaktiven Gruppe markiert [20-22].

Kürzlich hat es die Verwendung von Glas als Substrat und Fluoreszenz zur Detektion zusammen mit der Entwicklung neuer Technologien für die Synthese und für das Aufbringen der Nukleinsäuren in sehr hohen Dichten erlaubt, die Nukleinsäurearrays zu miniaturisierten bei gleichzeitiger Erhöhung des experimentellen Durchsatzes und des Informationsgehaltes [23-25].

Weiterhin ist aus WO 03/002763 bekannt, dass die Messung der Genexpression mittels Microarrays grundsätzlich für die Diagnose von Sepsis und sepsisähnlichen Zuständen verwendet werden können.

Eine Begründung für die Anwendbarkeit der Microarray-Technologie wurde zunächst durch klinische Untersuchungen auf dem Gebiet der Krebsforschung geliefert. Hier haben Expressionsprofile ihre Nützlichkeit bei der Identifizierung von Aktivitäten einzelner Gene oder Gengruppen gezeigt, die mit bestimmten klinischen Phänotypen korrelieren [26]. Durch die Analyse vieler Proben, die von Individuen mit oder ohne akute Leukämie oder diffusen B-Zell Lymphomen stammten, wurden Genexpressionsmarker (RNA) gefunden und anschließend für die klinisch relevante Klassifizierung dieser Krebsarten angewandt [26,27]. Golub et al. haben herausgefunden, daß verlässliche Vorhersagen nicht aufgrund von irgendeinem einzelnen Gen gemacht werden können, aber daß Vorhersagen, die auf der Veränderung der Transkritiption von 53 Genen (ausgewählt aus über 6000 Genen, die auf den Arrays vertreten waren) basieren, sehr genau sind [26].

Alisadeh et al. [27] untersuchten große B-Zell Lymphome (DLBCL). Die Autoren erarbeiteten Expressionsprofile mit einem "Lymphochip", einem Microarray, der 18 000 Klone komplementärer DNA trug und entwickelt worden war, um Gene zu überwachen, die in normale und abnormale Lymphozytenentwicklung involviert sind. Unter Anwendung von Cluster-Analysen waren sie in der Lage, DLBCL in zwei Kategorien einzuteilen, welche starke Unterschiede bezüglich der Überlebenschancen der Patienten aufzeigten. Die Genexpressionsprofile dieser Untergruppen entsprachen zwei bedeutsamen Stadien der B-Zelldifferenzierung.

Die grundsätzliche Verwendbarkeit von Genexpressionsprofilen, welche beispielsweise mittels der Microarray-Technik erhalten werden können, zur Diagnose von SIRS, generalisierten inflammatorischen Entzündungen, Sepsis und schwerer Sepsis ist in den nicht vorveröffentlichten deutschen Patentanmeldungen der Anmelderin der vorliegenden Erfindung DE 103 40 395.7, DE 103 36 511.7, DE 103 150 31.5 sowie 10 2004 009 952.9, auf die hiermit vollinhaltlich Bezug genommen wird, beschrieben.

Von Feezor et al. [28] ist bekannt, dass sich die Genexpressionen von Patienten, welche aufgrund ihrer operativen Behandlung ein SIRS mit Multiorgan Dysfunction Syndrome (MODS) entwickelten, gegenüber Patienten, die unter den gleichen operativen Bedingungen eine SIRS ohne MODS entwickelten, unterscheiden. Diese Untersuchungen lassen jedoch keine Aussage über die Unterscheidung von nichtinfektiösen MOV gegenüber infektiösen MOV zu, da in bei den Patienten keine Infektion nachgewiesen wurde.

Prucha et al. [30] beschäftigt sich mit der vergleichenden Genexpressionsanalyse in Bezug auf eine infektiösen Sepsis.

Heller et al. [31] offenbart eine Genexpressionsanalyse in Bezug auf die rheumatoide Arthritis.

Von "Details for HG-U95AV2:2024_S_AT" [33] ist ein Affymetrix-Chip bekannt, der zur Bestimmung von Genexpression verwendet werden kann und LYN-Gen Proben enthält.

Haslinger et al. [33] beschreibt ein Genexpressionsprofiling anhand von Blutproben von Patienten mit B-Zell chronische lymphatische Leukämie.

Die Verwendung von Genexpressionsprofilen zur Unterscheidung zwischen einem nichtinfektiösen MOV und einem infektiösen MOV wurde noch nicht beschrieben.

Ausgangspunkt für die in der vorliegenden Patentanmeldung offenbarten Erfindung ist die Erkenntnis, daß sich die Genexpressionen von Patienten mit nichtinfektiösem MOV von Genexpressionen von Patienten mit infektiösem MOV unterscheiden. Diese Unterschiede der Genexpressionen lassen es somit zu, anhand der Genexpression zwischen nichtinfektiösen und infektiösen MOV zu unterscheiden. Diese Unterscheidung ist mit den bisher zur Diagnose verwendeten klinischen Parametern nicht möglich, aber für die Einleitung einer spezialisierten intensivmedizinischen Therapie sehr bedeutungsvoll.

Der vorliegenden Erfindung liegt somit die Aufgabe zugrunde, durch die Verwendung von Genexpressionsmarkern zwischen einem nichtinfektiösen MOV und einem infektiösen MOV zu unterscheiden.

Diese Aufgabe wird durch die Merkmale der Ansprüche 1 gelöst.

Insbesondere betrifft die vorliegende Erfindung die Verwendung von in vitro aus einer Patientenprobe erhaltenen Genexpressionsprofilen für die Unterscheidung zwischen nichtinfektiösen und infektiösen Ursachen eines Multiorganversagens.

Die vorliegende Erfindung ist ferner nützlich als Ein- oder Ausschlußkriterium von Patienten, die an nichtinfektiösen oder infektiösen Ursachen eines Multiorganversagens erkrankt sind, in klinische Studien der Phasen 2-4.

Eine bevorzugte Ausführungsform der Erfindung liegt in der Erstellung von Genexpressionsdaten für die elektronische Weiterverarbeitung sowie zur Herstellung von Software für die Beschreibung der individuellen Prognose eines Sepsispatienten, für Diagnosezwecke und/oder Patientendatenmangementsystemen.

Die vorliegende Erfindung kann auch zur Herstellung von "in silico" Expertensystemen und/oder zur "in silico"-Modellierung von zelluläreren Signalübertragungswegen verwendet werden.

Zur Erstellung des Genexpressionsprofiles gemäß der vorliegenden Erfindung wird eine Mehrzahl von spezifischen Genen und/oder Genfragmenten verwendet, welche ausgewählt werden aus der Gruppe bestehend aus SEQ-ID No. 1 bis SEQ-ID No. 1297 sowie Genfragmenten davon mit wenigstens 20-200, bevorzugt 20-80 Nukleotiden.

Im Vergleich zu den Patienten mit nichtinfektiösem MOV weisen die aus der Gruppe bestehend aus: SEQ-ID No. 1 bis SEQ-ID No. 721 ausgewählten Gene und/oder Genfragmente bei Patienten mit infektiösem MOV signifikant gesteigerte Expressionswerte auf.

Im Vergleich zu den Patienten mit nichtinfektiösem MOV weisen die aus der Gruppe bestehend aus: SEQ-ID No. 722 bis SEQ-ID No. 1297 ausgewählten Gene und/oder Genfragmente bei Patienten mit infektiösem MOV signifikant reduzierte Expressionswerte auf.

Diese Sequenzen mit der Sequenz ID: 1 bis zur Sequenz ID: 1297 sind durch den Umfang der vorliegenden Erfindung mit umfaßt und sind dem angefügten, 1297 Sequenzen umfassenden, Sequenzprotokoll, das somit Bestandteil der Beschreibung der vorliegenden Erfindung ist, im Einzelnen offenbart und ist somit ebenfalls Bestandteil der Offenbarung der Erfindung. Dieses Sequenzprotokoll beinhaltet zudem eine Zuordnung der einzelnen Sequenzen mit der Sequenz ID: 1 bis zur Sequenz ID: 1297 zu deren GenBank Accession Nr. (Internet-Zugang über http://www.ncbi.nlm.nih.gov/).

Ferner kann man bei Patienten, die an nichtinfektiösen oder infektiösen Ursachen eines Multiorganversagens erkrankt sind, in klinische Studien der Phasen 2-4 die Genexpressionen in einer biologischen Flüssigkeit bestimmen und aus deren "Wert" Schlüsse hinsichtlich des Krankheitsverlaufs, der Überlebenswahrscheinlichkeit, des Therapieverlaufs oder der Ein- oder Ausschlussmöglichkeit dieser Patienten für klinische Studien ziehen.

Eine weitere Ausführungsform der Erfindung ist dadurch gekennzeichnet, daß die Genexpressionen mittels Hybridisierungsverfahren bestimmt wird.

Eine weitere Ausführungsform der Erfindung ist dadurch gekennzeichnet, daß die Genexpression mittels Microarrays bestimmt wird.

Eine weitere Ausführungsform der Erfindung ist dadurch gekennzeichnet, daß die Probe ausgewählt wird aus: Körperflüssigkeiten, insbesondere Blut, Liquor, Urin, Ascitesflüssigkeit, Seminalflüssigkeit, Speichel, Punktat; Zellinhalt oder eine Mischung davon.

Eine weitere Ausführungsform der Erfindung ist dadurch gekennzeichnet, daß Zellproben gegebenenfalls einer lytischen Behandlung unterzogen werden, um deren Zellinhalte freizusetzen.

Es ist dem Fachmann klar, daß die in den Ansprüchen dargelegten einzelnen Merkmale der Erfindung ohne Einschränkung beliebig miteinander kombinierbar sind.

Als Markergene im Sinne der Erfindung werden alle abgeleiteten DNA-Sequenzen, Partialsequenzen und synthetischen Analoga (beispielsweise Peptido-Nukleinsäuren, PNA) verstanden. Die auf Bestimmung der Genexpression auf RNA-Ebene bezogene Beschreibung der Erfindung stellt keine Einschränkung sondern nur eine beispielhafte Anwendung dar.

Die auf Blut bezogene Beschreibung der Erfindung stellt nur eine beispielhafte Anwendung der Erfindung dar. Als biologische Flüssigkeiten im Sinne der Erfindung werden alle Körperflüssigkeiten des Menschen verstanden.

Weitere Vorteile und Merkmale der vorliegenden Erfindung ergeben sich aufgrund der Beschreibung eines Ausführungsbeispiels, sowie anhand der Zeichnung.

Fig. 1 zeigt den pathologischen Verlauf von Multiorganversagen, ausgehend von unterschiedlichen medizinischen Zuständen.

### Ausführungsbeispiel

Untersuchungen der differentiellen Genexpression zur Unterscheidung zwischen nichtinfektiösen und infektiösen Ursachen eines Multiorganversagens.

Für die Messung der differentiellen Genexpression zur Unterscheidung zwischen nichtinfektiösen und infektiösen Ursachen eines Multiorganversagens wurden Untersuchungen von Vollblutproben von insgesamt 57 Patienten, welche auf operativen Intensivstationen behandelt wurden, durchgeführt.

Es wurden Vollblutproben von 31 Patienten abgenommen, welchen im Rahmen ihrer intensivmedizinischen Betreuung ein infektiöses MOV [dann als schwere Sepsis oder septischer Schock bezeichnet und klassifiziert nach 8] entwickelten.

Weiterhin wurden Vollblutproben von 26 Patienten abgenommen, welche im Rahmen ihrer intensivmedizinischen Betreuung ein nichtinfektiöses MOV [klassifiziert nach 8] entwickelten.

Als Referenzproben dienten die totale RNA aus Zelllinien SIG-M5.

Ausgewählte Charakteristika der beiden Patientengruppen sind in der Tabelle 1 dargestellt. Dabei werden Angaben zum Alter, Geschlecht, und dem SOFA-Score als Maß für die Funktion der Organsysteme gemacht. Gleichfalls sind die Plasmaproteinspiegel von Procalcitonin (PCT) und CRP, die Zahl der Leukozyten sowie die häufigsten CDC-Kriterien (Center of Disease Control) der Patienten angegeben.

Alle Patientenproben wurden mit der Referenzprobe jeweils auf einem Microarray ko-hybridisiert.

**Tabelle1: Daten der Patientengruppen**

| | **Patienten mit infektiösem MOV** | **Patienten mit nichtinfektiösem MOV** |
|---|---|---|
| **Anzahl** | 31 | 26 |
| **Geschlecht m/w** | 17/14 | 15/11 |
| **Alter*** [Jahre] | 60 (17) | 69 (10) |
| **APACHE-II-Score*** [Punkte] | 14 (10) | 14,9 (3,4) |
| **SOFA-Score*** [Punkte] | 10 (3) | 8 (3) |
| **Anzahl von OD*** | 3 (1) | 3 (1) |
| **PCT*** [ng/ml] | 3,1 (7,7) | 3.8 (6.7) |
| **CRP*** [µg/l] | 188 (168) | 80.2 (90.2) |
| **Leukozyten*** [Anz./l] | 13.00 (8 150) | 12.300 (6925) |
| **Art der Infektion It. CDC Kriterium: *Pneumonie*** | 15 Patienten | |
| ***Intraabdominelle Infektion*** | 13 Patienten | keine |
| ***Infektion OP-Ort ohne Wundinfektion*** | 2 Patienten | |
| ***Infektion des Gastrointestinaltrakt*** | 1 Patient | |
| * Median (Intraquartilabstand) | | |

### Experimentelle Beschreibung:

Nach Abnahme des Vollblutes wurde die totale RNA der Proben unter Anwendung des PAXGene Blood RNA Kit gemäß den Vorgaben des Herstellers (Qiagen) isoliert.

### Zellkultivierung

Für die Zellkultivierung (Kontrollproben) wurden 19 Kryozellkulturen (SIGM5) (eingefroren in flüssigem Stickstoff) genutzt. Die Zellen wurden jeweils mit 2 ml Iscove's Medium (Biochrom AG) beimpft ergänzt mit 20% fetalen Kälber Serum (FCS). Die Zellkulturen wurden anschliessend für 24 Stunden bei 37° C unter 5% CO2 in 12-well Platten inkubiert. Danach wurde der Inhalt von 18 Wells in 2 Teile mit jeweils dem gleichen Volumen geteilt, sodass schliesslich 3 Platten des gleichen Formats (insgesamt 36 Wells) zur Verfügung standen. Die Kultivierung wurde anschliessend für 24 Stunden unter den gleichen Bedingungen fortgeführt. Im Anschluss daran wurden die resultierenden Kulturen von 11 Wells jeder Platte vereint und zentrifugiert (1000 x g, 5 min, Raumtemperatur). Der Überstand wurde verworfen und das Zellpellet in 40 ml des o.g. Mediums gelöst. Diese 40 ml gelöste Zellen wurden in zwei 250 ml Kolben zu gleichen Teilen aufgeteilt und nach 48 Stunden Inkubation und Zugabe von 5 ml des o.g. Mediums wiederum inkubiert. Von den restlichen 2 ml der zwei verbleibendenden Platten wurden 80 µl in leere Wells der gleichen Platten gegeben, welche bereits vorher mit 1 ml des o.g. Mediums präpariert waren. Nach 48 Stunden Inkubation wurde nur eine der 12 Well-Platten wie folgt prozessiert: Aus jedem Well wurden 500 µl entnommen und vereint. Die daraus resultierenden 6 ml wurden in einen 250 ml Kolben gegeben, welcher ca. 10 ml frisches Medium enthielt. Dieses Gemisch wurde mit 1000 x g 5 Minuten bei Raumtemperatur zentrifugiert und in 10 ml des o.g. Mediums gelöst. Die anschliessende Zellzählung ergab folgendes Ergebnis: 1,5 x 107 Zellen pro ml, 10 ml Gesamtvolumen, Gesamtzahl der Zellen: 1,5 x 108. Da die Zellzahl noch nicht ausreichend war, wurden 2,5 ml des o.g. Zellsuspension in 30 ml des o.g. Mediums in einen 250 ml (75 cm²) Kolben gegeben (insgesamt 4 Kolben). Nach 72 Sunden Inkubationszeit wurden jeweils 20 ml frischen Mediums in die Kolben gegeben. Nach folgender 24-stündiger Inkubation erfolgte die Zellzählung wie oben beschrieben, die eine Gesamtzellzahl von 3,8 x 10⁸ Zellen ergab. Um die gewünschte Zellzahl von 2 x 106 Zellen zu errreichen wurden die Zellen in 47,5 ml des o.g. Mediums in 4 Kolben resuspendiert. Nach einer Inkubationszeit von 24 Stunden wurden die Zellen zentrifugiert und zweimal mit Phosphatpuffer ohne Ca²⁺ und Mg²⁺ (Biochrom AG) gewaschen.

Die Isolation der totalen RNA erfolgt mittels des NucleoSpin RNA L Kits (Machery&Nagel) entsprechend den Angaben des Herstellers. Die oben beschriebene Prozedur wurde wiederholt bis die erforderliche Zellzahl erreicht wurde. Dies war erforderlich, um die erforderliche Menge von 6 mg totale RNA zu erreichen, was etwa einer Effizienz von 600 µg RNA pro 108 Zellen entspricht.

### Reverse Transkription / Markierung / Hybridisierung

Nach Abnahme des Vollblutes wurde die totale RNA der Proben unter Verwendung des PAXGene Blood RNA Kits (PreAnalytiX) gemäss den Vorgaben des Herstellers isoliert und auf ihre Qualität geprüft. Von jeder Probe wurden 10 µg totale RNA aliquotiert und zusammen mit 10 µg total RNA aus SIGM5-Zellen als Referenz-RNA zu komplementärer DNA (cDNA) mit der reversen Transkriptase Superscript II (Invitrogen) umgeschrieben und die RNA anschließend durch alkalische Hydrolyse aus dem Ansatz entfernt. Im Reaktionsansatz wurde ein Teil des dTTP durch Aminoallyl-dUTP (AA-dUTP) ersetzt, um später die Kopplung des Fluoreszenzfarbstoffes an die cDNA zu ermöglichen.

Nach der Aufreinigung des Reaktionsansatzes wurden die cDNA der Proben und Kontrollen mit den Fluoreszenzfarbstoffen Alexa 647 und Alexa 555 kovalent markiert und auf einem Microarray der Firma SIRS-Lab hybridisiert. Auf dem verwendeten Microarray befinden sich 5308 immobilisierte Polynukleotide mit einer Länge von 55 - 70 Basenpaaren, die jeweils ein humanes Gen repräsentieren und Kontrollspots zur Qualitätssicherung. Ein Microarray unterteilt sich in 28 Subarrays mit einem Raster von 15x15 Spots.

Die Hybridisierung und das anschliessende Waschen bzw. Trocknen wurde in der Hybridisierungsstation HS 400 (Tecan) nach Angaben des Herstellers über 10,5 Stunden bei 42 °C durchgeführt. Die verwendete Hybridisierungslösung besteht aus den jeweiligen gelabelten cDNA-Proben, 3,5x SSC (1x SSC enthält 150 mM Natriumchlorid und 15 mM Natriumcitrat), 0,3% Natriumdodecylsulfat (V/V) 25% Formamid (V/V) und je 0,8 µg µl-1 cot-1 DNA, Hefe t-RNA und poly-A RNA. Das anschliessende Waschen der Mikroarrays wurde mit nachfolgendem Programm bei Raumtemperatur in durchgeführt: je 90 Sekunden spülen mit Waschpuffer 1 (2x SSC, 0,03% Natriumdodecylsulfat), mit Waschpuffer 2 (1x SSC) und abschließend mit Waschpuffer 3 (0,2x SSC). Danach wurden die Mikroarrays unter einem Stickstoffstrom mit einem Druck von 2,5 bar bei 30 °C über 150 Sekunden getrocknet.

Nach der Hybridisierung wurden die Hybridisierungssignale der Mikroarrays mit einem GenePix 4000B Scanner (Axon) ausgelesen und die Expressionsverhältnisse der differenziert exprimierten Gene mit der Software GenePix Pro 4.0 (Axon) bestimmt.

### Auswertung:

Für die Auswertung wurde die mittlere Intensität eines Spots als der Medianwert der zugehörigen Spotpixel bestimmt.

### Korrektur systematischer Fehler:

Die Korrektur systematischer Fehler erfolgte nach dem Ansatz von Huber et al. (2002). Dabei wurden der additive und der multiplikative Bias innerhalb eines Microarrays aus 70% der vorhandenen Genproben geschätzt. Für alle weiteren Berechnungen wurden die Signale mittels arcus sinus hyperbolicus transformiert.

Für die Analyse wurden die normalisierten und transformierten relativen Verhältnisse der Signale der Patientenproben gegen die allgemeine Kontrolle berechnet. D.h. für das j-te Gen des n-ten Patienten ergab die Berechnung den Wert Gj,n = arcsinh(Scy5(j,n)) - arcsinh(Scy3(j,n)), wobei [SCy3(j,n), SCy5(j,n)] das zugehörige Signalpaar bezeichnet. War für einen Patienten ein Spot nicht auswertbar (z.B. Fleck auf dem gescannten Bild), so wurde der zugehörige Wert als nicht vorhanden (,missing value') gekennzeichnet.

### Statistischer Vergleich:

Für den Vergleich wurde der zweiseitige Zweistichproben Student-Test pro Gen verwendet. Beide Stichproben enthielten die Werte der Patientengruppen nichtinfektiöses MOV bzw. infektiöses MOV. Für die Auswahl der differenziert exprimierten Gene wurde der zugehörige p-Wert bewertet. Für die Gruppe der ausgewählten Gene war der zugehörige p-Wert kleiner als 0.05.

Die Höhe des Expressionsverhältnisses jedes Gens stellte das Kriterium für eine Sortierung der untersuchten Gene dar. Von Interesse waren die Gene, die zwischen den an einem nichtinfektiösen MOV erkrankten Patienten gegenüber den an infektiösen MOV erkrankten Patienten am meisten überexprimiert bzw. unterexprimiert wurden.

Aus Tabelle 2 ist ersichtlich, dass 721 Gene den Patientenprobe gefunden wurden, die in den Patienten mit infektiösem MOV gegenüber den Patienten mit nichtinfektiösem MOV signifikant überexprimiert waren. Weiterhin ist aus Tabelle 3 ersichtlich, dass 576 Gene der Patienten mit infektiösem MOV gegenüber den Patienten mit nichtinfektiösem MOV signifikant unterexprimiert waren. Aus den Ergebnissen wird deutlich, dass die in Tabelle 2 und Tabelle 3 aufgeführten Genexpressionen zwischen den nichtinfektiösen Ursachen eines Multiorganversagen und den infektiösen Ursachen eines Multiorganversagen (unterscheiden. Somit stellen die aufgeführten Genexpressionen Marker für eine Unterscheidung von nichtinfektiösen und infektiösen Ursachen eines Multiorganversagens dar.

**Tabelle 2: Signifikant gesteigerte Genexpressionen in Proben von Patienten mit infektiösem MOV, im Vergleich zu den Genexpressionen von Patienten mit nichtinfektiösem MOV**

| **GenBank Accession-Number** | **p-Wert** | **Mittlerer normalisierter und transformierter Expressionswert** | | **Standardabweichung** | | **Sequenz-ID** |
|---|---|---|---|---|---|---|
| | | **Gruppe der Patienten mit nichtinfektiösem /MOV** | **Gruppe der Patienten mit Infektiösem MOV** | **Gruppe der Patienten mit nichtinfektiösem /MOV** | **Gruppe der Patienten mit infektiösem MOV** | |
| N32857 | 0,00 | -2,99 | 0,20 | 1,42 | 2,78 | 1 |
| N32853 | 0,00 | -0,85 | 1,60 | 2,15 | 2,89 | 2 |
| N32495 | 0,00 | -2,38 | -0,56 | 1,37 | 0,40 | 3 |
| AI701077 | 0,01 | -0,33 | 1,46 | 0,17 | 3,08 | 4 |
| M87790 | 0,00 | 1,18 | 2,93 | 1,13 | 1,24 | 5 |
| AI559317 | 0,01 | 0,20 | 1,83 | 0,54 | 2,60 | 6 |
| N34897 | 0,00 | -2,60 | -1,05 | 1,61 | 0,54 | 7 |
| AA907084 | 0,02 | 0,53 | 1,94 | 0,49 | 2,58 | 8 |
| N45223 | 0,00 | -2,88 | -1,54 | 1,24 | 0,57 | 9 |
| H70430 | 0,03 | 0,12 | 1,42 | 0,70 | 2,73 | 10 |
| R59591 | 0,01 | -0,25 | 0,97 | 0,20 | 2,06 | 11 |
| N47688 | 0.00 | -2,47 | -1,27 | 0,94 | 0,44 | 12 |
| N52930 | 0,00 | -1,49 | -0,30 | 1,06 | 0,76 | 13 |
| XM_004256 | 0,00 | -3,10 | 1,94 | 0,62 | 1,43 | 14 |
| AJ010446 | 0,00 | -0,22 | 0,93 | 0,66 | 1,11 | 15 |
| N35225 | 0,00 | -2,81 | -1,75 | 1,21 | 0,52 | 16 |
| N50680 | 0,00 | -1,30 | -0,29 | 1,58 | 0,46 | 17 |
| BC018761 | 0,00 | 1,04 | 2,02 | 0,80 | 1,27 | 18 |
| XM_009475 | 0,00 | -2,54 | -1,58 | 0,83 | 0,91 | 19 |
| N53369 | 0,04 | -0,37 | 0,55 | 1,62 | 1.39 | 20 |
| AI420863 | 0,05 | -0,17 | 0,74 | 0,47 | 1,99 | 21 |
| N33423 | 0,05 | -0,32 | 0,58 | 1,64 | 1,51 | 22 |
| AA843281 | 0,05 | 0,27 | 1,15 | 0,54 | 1,88 | 23 |
| X64641 | 0,02 | 0,26 | 1,11 | 1,09 | 1,23 | 24 |
| N52545 | 0,00 | -1,10 | -0,28 | 1,02 | 0,55 | 25 |
| X57817 | 0,01 | 0,19 | 1,00 | 0,58 | 1,21 | 26 |
| N58236 | 0,00 | -0,68 | 0,14 | 0,85 | 0,56 | 27 |
| XM_056556 | 0,00 | -3,12 | -2,31 | 0,58 | 0,99 | 28 |
| N59170 | 0,01 | -0,22 | 0,59 | 1,35 | 0,74 | 29 |
| N58392 | 0,00 | -0,85 | -0,04 | 0,71 | 0,62 | 30 |
| N34672 | 0,02 | -0,54 | 0,26 | 1,74 | 0,39 | 31 |
| XM_015396 | 0,00 | -0,27 | 0,52 | 0,68 | 0,81 | 32 |
| X05875 | 0.01 | -2,86 | -2,09 | 0,55 | 1,15 | 33 |
| N48715 | 0,00 | -1,12 | -0,36 | 0,70 | 0,61 | 34 |
| N90140 | 0,05 | -0,44 | 0,32 | 0,29 | 1,71 | 35 |
| NM_002415 | 0,00 | -1,66 | -0,90 | 0,63 | 0,56 | 36 |
| AI890242 | 0,00 | -0,14 | 0,59 | 0,25 | 0,72 | 37 |
| AI589096 | 0,00 | -0,39 | 0,32 | 0,56 | 0,54 | 38 |
| NM_001911 | 0,04 | -2,61 | -1,91 | 0,77 | 1,44 | 39 |
| N39242 | 0,05 | -0,56 | 0,12 | 1,75 | 0,52 | 40 |
| N35493 | 0,04 | -0,45 | 0,23 | 1,69 | 0,46 | 41 |
| AI271764 | 0,00 | -0,66 | -0,02 | 0,70 | 0,62 | 42 |
| NM_006936 | 0,00 | -2,00 | -1,37 | 0,46 | 0,55 | 43 |
| NM_005225 | 0,00 | -0,90 | -0,26 | 0,58 | 0,53 | 44 |
| R98960 | 0,04 | -0,37 | 0,26 | 1,41 | 0,70 | 45 |
| NM_000714.3 | 0,00 | 0,48 | 1,11 | 0,44 | 0,88 | 46 |
| N48180 | 0,01 | -1,08 | -0,45 | 1,05 | 0,45 | 47 |
| NM_002295 | 0,02 | -3,17 | -2,56 | 0,44 | 1,09 | 48 |
| AI697365 | 0,01 | 0,62 | 1,22 | 0,82 | 0,67 | 49 |
| NM_001404 | 0,00 | -2,56 | -1,96 | 0,38 | 0,86 | 50 |
| NM_176800.1 | 0,00 | -0,31 | 0,29 | 0,51 | 0,42 | 51 |
| XM_027885 | 0,03 | -3,23 | -2,63 | 0,37 | 1,17 | 52 |
| NM_006597.3 | 0,00 | -2,42 | -1.84 | 0,50 | 0,79 | 53 |
| NM_002211 | 0,00 | -1,59 | -1,02 | 0,68 | 0,54 | 54 |
| NM_001570 | 0,00 | -0,55 | 0,03 | 0,53 | 0,49 | 55 |
| AI888606 | 0,03 | -0,16 | 0,40 | 0,44 | 1,11 | 56 |
| NM_006636.2 | 0,04 | -3,49 | -2,93 | 0,53 | 1,16 | 57 |
| AA458827 | 0,00 | 0,15 | 0,71 | 0,33 | 0,60 | 58 |
| AA398757 | 0,01 | 0,11 | 0,67 | 0,57 | 0,81 | 59 |
| NM_000814.2 | 0,00 | -0,07 | 0,49 | 0,48 | 0,75 | 60 |
| NM_000963 | 0,00 | -0,41 | 0,15 | 0,86 | 0,33 | 61 |
| AI913322 | 0,02 | -0,68 | -0,14 | 0,68 | 0,92 | 62 |
| N20922 | 0,04 | -0,72 | -0,17 | 1,27 | 0,51 | 63 |
| R49085 | 0,00 | 0,02 | 0,57 | 0,64 | 0,56 | 64 |
| N54935 | 0,01 | -0,74 | -0,19 | 0,97 | 0,38 | 65 |
| XM_027358 | 0,01 | -1,49 | -0,95 | 0,75 | 0,59 | 66 |
| NM_031200 | 0,00 | 0,11 | 0,65 | 0,57 | 0,57 | 67 |
| AA805531 | 0,00 | -0,07 | 0,47 | 0,33 | 0,53 | 68 |
| NM_000194 | 0,04 | -2,64 | -2,12 | 0,70 | 1,02 | 69 |
| AI623567 | 0,01 | 0,39 | 0,92 | 0,59 | 0,73 | 70 |
| N64495 | 0,00 | -0,49 | 0,02 | 0,63 | 0,37 | 71 |
| NM_002156 | 0,01 | -2,26 | -1,75 | 0,59 | 0,70 | 72 |
| NM_012068 | 0,00 | -1,40 | -0,89 | 0,54 | 0,40 | 73 |
| R43722 | 0,02 | -0,45 | 0,05 | 0,65 | 0,80 | 74 |
| NM_001686 | 0,03 | -2,63 | -2,13 | 0,32 | 0,93 | 75 |
| NM_002969 | 0,00 | -0,92 | -0,42 | 0,46 | 0,53 | 76 |
| NM_003295 | 0,04 | -2,72 | -2,24 | 0,45 | 0,98 | 77 |
| XM_039372 | 0,02 | -2,43 | -1,95 | 0,26 | 0,92 | 78 |
| AA731679 | 0,02 | 0,17 | 0,65 | 0,79 | 0,61 | 79 |
| AA620762 | 0,00 | -0,04 | 0,44 | 0,21 | 0,50 | 80 |
| AI499889 | 0,01 | -0,01 | 0,47 | 0,67 | 0,64 | 81 |
| N33530 | 0,00 | -0,30 | 0,18 | 0,70 | 0,31 | 82 |
| NM_002033 | 0,00 | -1,92 | -1,44 | 0,39 | 0,63 | 83 |
| AA436651 | 0,00 | -0,26 | 0,21 | 0,54 | 0,26 | 84 |
| NM_001540 | 0,00 | -1,42 | -0,95 | 0,42 | 0,54 | 85 |
| NM_004257 | 0,00 | -0,85 | -0,38 | 0,33 | 0,25 | 86 |
| NM_014280.1 | 0,00 | -1,45 | -0,98 | 0,58 | 0,47 | 87 |
| NM_000930.2 | 0,00 | -1,30 | -0,83 | 0,64 | 0,51 | 88 |
| XM_002101 | 0,00 | -0,63 | -0.17 | 0,63 | 0,27 | 89 |
| AI733269 | 0,00 | -0,18 | 0,29 | 0,45 | 0,36 | 90 |
| NM_001168 | 0,02 | -2,14 | -1,67 | 0,61 | 0,77 | 91 |
| XM_052636 | 0,00 | -1,51 | -1,04 | 0,35 | 0,48 | 92 |
| AI689318 | 0,00 | -1,00 | -0,54 | 0,55 | 0,46 | 93 |
| NM_001212 | 0,01 | -1,65 | -1,19 | 0,56 | 0,64 | 94 |
| R37251 | 0,00 | 0,61 | 1,06 | 0,39 | 0,63 | 95 |
| NM_001166 | 0,00 | -0,76 | -0,31 | 0,53 | 0,41 | 96 |
| XM_056798 | 0,01 | -1,34 | -0,89 | 0,66 | 0,51 | 97 |
| NM_005052 | 0,01 | 0,41 | 0,86 | 0,37 | 0,67 | 98 |
| NM_003379 | 0,00 | -1,45 | -1,00 | 0,41 | 0,51 | 99 |
| XM_048068 | 0,00 | -0,37 | 0,08 | 0,43 | 0,42 | 100 |
| NM_000577 | 0,01 | 0,46 | 0,90 | 0,32 | 0,67 | 101 |
| NM_001101 | 0,00 | -0,69 | -0,25 | 0,43 | 0,58 | 102 |
| D31890 | 0,01 | -1,79 | -1,36 | 0,56 | 0,55 | 103 |
| N49976 | 0,03 | -0,26 | 0,17 | 0,90 | 0,50 | 104 |
| XM_008679 | 0,01 | -0,85 | -0,41 | 0,57 | 0,56 | 105 |
| N33187 | 0,01 | -0,06 | 0,38 | 0,52 | 0,54 | 106 |
| R42782 | 0,00 | -0,09 | 0,34 | 0,36 | 0,45 | 107 |
| N49751 | 0,01 | 0,71 | 1,14 | 0,48 | 0,64 | 108 |
| AI910456 | 0,04 | -1,12 | -0,69 | 0,73 | 0,75 | 109 |
| NM_001569 | 0,00 | -1,10 | -0,67 | 0.38 | 0,46 | 110 |
| H90322 | 0,00 | 0,05 | 0,48 | 0,27 | 0,51 | 111 |
| AI926659 | 0,00 | 0,05 | 0,48 | 0,37 | 0,44 | 112 |
| XM_047499 | 0,01 | -1,29 | -0,86 | 0,46 | 0,67 | 113 |
| AA437224 | 0,00 | -0,71 | -0,28 | 0,46 | 0,24 | 114 |
| NM_021798 | 0,00 | -0,32 | 0,11 | 0,44 | 0,36 | 115 |
| NM_000584 | 0,02 | -1,82 | -1,40 | 0,61 | 0,64 | 116 |
| AA452122 | 0,00 | -0,40 | 0,02 | 0,60 | 0,41 | 117 |
| NM_002189 | 0,01 | 0,10 | 0,52 | 0,48 | 0,57 | 118 |
| AA001367 | 0,00 | -0,13 | 0,29 | 0,37 | 0,57 | 119 |
| AI129679 | 0,00 | -1,27 | -0,85 | 0,31 | 0,37 | 120 |
| D26599 | 0,01 | -1,90 | -1,48 | 0,50 | 0,58 | 121 |
| NM_170665.2 | 0,00 | -1,19 | -0,78 | 0,49 | 0,45 | 122 |
| NM_006419 | 0,00 | -0,16 | 0,25 | 0,39 | 0,51 | 123 |
| W85706 | 0,00 | -1,07 | -0,66 | 0,30 | 0,37 | 124 |
| AA897528 | 0,00 | -0,50 | -0,09 | 0,65 | 0,30 | 125 |
| NM_003358 | 0,04 | 0,56 | 0,97 | 0,50 | 0,82 | 126 |
| N35251 | 0,00 | -0,18 | 0,22 | 0,52 | 0,41 | 127 |
| NM_004863 | 0,00 | -0,63 | -0,22 | 0,37 | 0,48 | 128 |
| NM_001950 | 0,00 | -0,82 | -0,41 | 0,40 | 0,33 | 129 |
| NM_006260 | 0,03 | -0,63 | -0,22 | 0,61 | 0,67 | 130 |
| NM_170708 | 0,03 | -1,52 | -1,12 | 0,54 | 0,63 | 131 |
| N63024 | 0,01 | 0,64 | 1,04 | 0,43 | 0,56 | 132 |
| NM_017595 | 0,00 | -0,85 | -0,45 | 0,36 | 0,33 | 133 |
| A1364529 | 0,02 | -0,97 | -0,57 | 0,59 | 0,59 | 134 |
| NM_013432 | 0,00 | -0,30 | 0,10 | 0,31 | 0,28 | 135 |
| NM_006736.2 | 0,00 | -0,56 | -0,16 | 0,24 | 0,37 | 136 |
| NM_002128 | 0,02 | -1,84 | -1,44 | 0,40 | 0,69 | 137 |
| AA441793 | 0,00 | -0,70 | -0,31 | 0,45 | 0,33 | 138 |
| N76019 | 0,00 | -0,27 | 0,13 | 0,35 | 0,30 | 139 |
| XM_048665 | 0,00 | -0,28 | 0,11 | 0,38 | 0,35 | 140 |
| NM_003467 | 0,01 | -1,80 | -1,41 | 0,32 | 0,62 | 141 |
| N59330 | 0,01 | -0,21 | 0,19 | 0,56 | 0,50 | 142 |
| NM_004672 | 0,00 | -0,08 | 0,32 | 0,44 | 0,26 | 143 |
| AA426021 | 0,01 | 0,06 | 0,45 | 0,28 | 0,61 | 144 |
| XM_008608 | 0,00 | -0,60 | -0,21 | 0,54 | 0,34 | 145 |
| H44908 | 0,00 | -0,55 | -0,16 | 0,42 | 0,33 | 146 |
| AA699412 | 0,00 | -0,47 | -0,08 | 0,48 | 0,35 | 147 |
| AI572080 | 0,01 | 0,28 | 0,67 | 0,41 | 0,52 | 148 |
| NM_012072 | 0,02 | -1,86 | -1,47 | 0,47 | 0,63 | 149 |
| XM_035638 | 0,04 | -1,96 | -1,57 | 0,40 | 0,80 | 150 |
| BC001604 | 0,00 | -1,13 | -0,74 | 0,40 | 0,33 | 151 |
| AA481282 | 0,00 | -0,11 | 0,27 | 0,54 | 0,40 | 152 |
| NM_003376 | 0,01 | -1,17 | -0,78 | 0,54 | 0,42 | 153 |
| H11661 | 0,00 | -0,07 | 0,32 | 0,25 | 0,37 | 154 |
| AI435179 | 0,01 | -0,08 | 0,30 | 0,68 | 0,37 | 155 |
| XM_006800 | 0,01 | 0,19 | 0,57 | 0,38 | 0,55 | 156 |
| NM_000397.2 | 0,00 | -0,56 | -0,17 | 0,37 | 0,28 | 157 |
| AA424023 | 0,02 | 0,01 | 0,39 | 0,43 | 0,63 | 158 |
| XM_012949 | 0,02 | -1,81 | -1,43 | 0,45 | 0,64 | 159 |
| W84866 | 0,00 | 0,14 | 0,52 | 0,44 | 0,45 | 160 |
| N62672 | 0,01 | -0,21 | 0,17 | 0,60 | 0.44 | 161 |
| NM_001530 | 0,01 | -0,16 | 0,21 | 0,25 | 0,62 | 162 |
| NM_002157.1 | 0,03 | -2,21 | -1,83 | 0,43 | 0,71 | 163 |
| NM_003258 | 0,02 | -1,80 | -1,43 | 0,68 | 0,46 | 164 |
| A1863135 | 0,04 | 0,87 | 1,25 | 0,40 | 0,76 | 165 |
| NM_004083 | 0,01 | -0,95 | -0,58 | 0,46 | 0,47 | 166 |
| H06194 | 0,00 | -0,92 | -0,54 | 0,45 | 0,30 | 167 |
| XM_047570 | 0,03 | -1,61 | -1,24 | 0,41 | 0,68 | 168 |
| D26598 | 0,01 | -1,23 | -0,86 | 0,27 | 0,57 | 169 |
| R44955 | 0,01 | -0,08 | 0,29 | 0,55 | 0,49 | 170 |
| NM_012297 | 0,02 | -1,60 | -1,22 | 0,48 | 0,59 | 171 |
| T84080 | 0,02 | 0,13 | 0,49 | 0,57 | 0,52 | 172 |
| H52810 | 0,00 | 0.13 | 0,50 | 0,33 | 0,44 | 173 |
| XM_055188 | 0,04 | 0,94 | 1,30 | 0,36 | 0,75 | 174 |
| AI184987 | 0,01 | 0,19 | 0,56 | 0,51 | 0,50 | 175 |
| AI733177 | 0,02 | 0,54 | 0.90 | 0,41 | 0,63 | 176 |
| NM_006016 | 0,02 | -1,15 | -0,78 | 0,44 | 0,60 | 177 |
| XM_006867 | 0,02 | 0,09 | 0,46 | 0,33 | 0,62 | 178 |
| NM_004475.1 | 0,02 | 0,95 | 1,32 | 0,40 | 0,60 | 179 |
| AA485242 | 0,03 | 0,34 | 0,70 | 0,49 | 0,62 | 180 |
| NM_003300 | 0,01 | -1,49 | -1,13 | 0,31 | 0,54 | 181 |
| NM_032957 | 0,00 | -0,88 | -0,52 | 0,32 | 0,39 | 182 |
| XM_033862 | 0,00 | -0,01 | 0,35 | 0,29 | 0,36 | 183 |
| W80385 | 0,01 | 0,10 | 0,46 | 0,36 | 0,52 | 184 |
| H99099 | 0,01 | -0,04 | 0,32 | 0,37 | 0,52 | 185 |
| N67859 | 0,00 | -0,77 | -0,41 | 0,34 | 0,39 | 186 |
| NM_001013 | 0,04 | -1,88 | -1,52 | 0,44 | 0,68 | 187 |
| NM_006641 | 0,02 | -0,36 | 0,00 | 0,69 | 0,35 | 188 |
| N70546 | 0,00 | -0,11 | 0,25 | 0,38 | 0,40 | 189 |
| XM_015278 | 0,00 | -0,36 | -0,01 | 0,33 | 0,42 | 190 |
| AI932670 | 0,00 | -0,15 | 0,20 | 0,36 | 0,43 | 191 |
| NM_175617 | 0,00 | -0,11 | 0,25 | 0,29 | 0,26 | 192 |
| NM_004377.2 | 0,02 | -0,88 | -0,53 | 0,52 | 0,50 | 193 |
| NM_003153 | 0,00 | -0,39 | -0,04 | 0,30 | 0,48 | 194 |
| AI910804 | 0,03 | -0,65 | -0,30 | 0,51 | 0,57 | 195 |
| AI221860 | 0,00 | -0,30 | 0,05 | 0,17 | 0,43 | 196 |
| AI866414 | 0,00 | -0,37 | -0,02 | 0,33 | 0,27 | 197 |
| BC020968 | 0,03 | -1,77 | -1,42 | 0,36 | 0,65 | 198 |
| AA484213 | 0,05 | -0,49 | -0,14 | 0.90 | 0,27 | 199 |
| XM_003593 | 0,00 | -0,52 | -0,17 | 0,44 | 0,27 | 200 |
| XM_008738 | 0,02 | -1,46 | -1,11 | 0,54 | 0,49 | 201 |
| NM_032964 | 0,00 | -0,48 | -0,13 | 0,41 | 0,20 | 202 |
| NM_001455 | 0,00 | 0,26 | 0,61 | 0,42 | 0,41 | 203 |
| NM_002994 | 0,00 | -0,61 | -0,26 | 0,35 | 0,43 | 204 |
| NM_004222 | 0,00 | -1,44 | -1,10 | 0,30 | 0,45 | 205 |
| H48923 | 0,00 | -0,59 | -0,25 | 0,35 | 0,39 | 206 |
| T47430 | 0,05 | 0,41 | 0,75 | 0,38 | 0,71 | 207 |
| NM_032963 | 0,00 | -0,45 | -0,11 | 0,52 | 0,22 | 208 |
| XM_045933 | 0,00 | 0,22 | 0,56 | 0,23 | 0,41 | 209 |
| T99746 | 0,03 | 0,26 | 0,60 | 0,49 | 0,52 | 210 |
| XM_012039 | 0,01 | -1,44 | -1,10 | 0,44 | 0,43 | 211 |
| NM_004740 | 0,00 | -0,46 | -0,12 | 0,29 | 0,24 | 212 |
| NM_001681.2 | 0,05 | -1,39 | -1,05 | 0,61 | 0,60 | 213 |
| AI027259 | 0,00 | -0,40 | -0,06 | 0,49 | 0,28 | 214 |
| AA431552 | 0,00 | -0,63 | -0,30 | 0,41 | 0,32 | 215 |
| NM_000029 | 0,00 | 0,30 | 0,63 | 0,32 | 0,42 | 216 |
| XM_041847 | 0,05 | -1,02 | -0,68 | 0,69 | 0,51 | 217 |
| NM_005920 | 0,00 | -0.90 | -0,56 | 0,28 | 0,33 | 218 |
| NM_002394 | 0,01 | -1,03 | -0,69 | 0,49 | 0,39 | 219 |
| Al093704 | 0,01 | -0,32 | 0,02 | 0,35 | 0,47 | 220 |
| XM_043359 | 0,01 | 0,21 | 0,55 | 0,36 | 0,51 | 221 |
| H48445 | 0,01 | 0,28 | 0,61 | 0,38 | 0,53 | 222 |
| XM_015815 | 0,02 | -1,13 | -0,80 | 0,52 | 0,50 | 223 |
| NM_001774 | 0,00 | -0,07 | 0,27 | 0,31 | 0,42 | 224 |
| Al937053 | 0,00 | -0,42 | -0,09 | 0,39 | 0,26 | 225 |
| AA493719 | 0,01 | -0.61 | -0,28 | 0,52 | 0,35 | 226 |
| NM_002996 | 0,01 | 0.19 | 0,51 | 0,33 | 0,44 | 227 |
| AI025039 | 0,01 | 0,16 | 0,49 | 0,31 | 0,47 | 228 |
| NM_139049 | 0,02 | -0,52 | -0,19 | 0,60 | 0,34 | 229 |
| NM_006238.2 | 0,00 | -0,29 | 0,04 | 0,21 | 0,26 | 230 |
| XM_031456 | 0,00 | -0,67 | -0,35 | 0,30 | 0,30 | 231 |
| AA455096 | 0,00 | -0,29 | 0,03 | 0,29 | 0,32 | 232 |
| XM_047675 | 0,03 | 0,36 | 0,68 | 0,26 | 0,65 | 233 |
| AI809252 | 0.00 | -0,14 | 0.18 | 0,36 | 0,34 | 234 |
| NM_139047 | 0,00 | -0,45 | -0,13 | 0,47 | 0,31 | 235 |
| AI760793 | 0,01 | -0,47 | -0,15 | 0,47 | 0,33 | 236 |
| NM 000204 | 0,00 | -0,03 | 0,29 | 0,25 | 0,40 | 237 |
| AI860121 | 0,01 | 0,55 | 0,87 | 0,37 | 0,48 | 238 |
| H50222 | 0,00 | -0,13 | 0,19 | 0,19 | 0,34 | 239 |
| XM_041101 | 0,02 | -1,06 | -0,74 | 0,34 | 0,56 | 240 |
| XM_035854 | 0,01 | 0,09 | 0,41 | 0,47 | 0,44 | 241 |
| AA043903 | 0,01 | -0,73 | -0,41 | 0,52 | 0,31 | 242 |
| R40406 | 0,03 | -0,89 | -0,58 | 0,48 | 0,52 | 243 |
| N98510 | 0,04 | -1,22 | -0,90 | 0,51 | 0,56 | 244 |
| H05449 | 0,03 | -0,15 | 0,16 | 0,52 | 0,50 | 245 |
| AI567338 | 0,01 | -0,11 | 0,20 | 0,43 | 0,39 | 246 |
| NM 000308.1 | 0,00 | -0,19 | 0,13 | 0,29 | 0,42 | 247 |
| R40880 | 0,00 | -0,21 | 0,11 | 0,40 | 0,34 | 248 |
| H52284 | 0,00 | -0,26 | 0,05 | 0,41 | 0,27 | 249 |
| NM_030662 | 0,00 | -0,47 | -0,16 | 0,22 | 0,27 | 250 |
| NM_032965 | 0,02 | 0,52 | 0,83 | 0,41 | 0.47 | 251 |
| NM_004322 | 0,00 | -0,34 | -0,03 | 0,32 | 0,27 | 252 |
| XM_002762 | 0,00 | -0,52 | -0,21 | 0,22 | 0,25 | 253 |
| AI679230 | 0,00 | -0,40 | -0,09 | 0,43 | 0,33 | 254 |
| AI368670 | 0,00 | -0,23 | 0,08 | 0,30 | 0,31 | 255 |
| NM_006415 | 0,01 | -0,71 | -0,40 | 0,45 | 0,33 | 256 |
| NM_004379 | 0,00 | -0,54 | -0,23 | 0,24 | 0,24 | 257 |
| NM_002974 | 0,02 | 0,02 | 0,33 | 0,49 | 0.38 | 258 |
| AI914729 | 0,02 | -0,11 | 0,20 | 0,51 | 0,40 | 259 |
| NM_032989 | 0,00 | -0,17 | 0,14 | 0,23 | 0,28 | 260 |
| AI799645 | 0,04 | 0,14 | 0,45 | 0,39 | 0,61 | 261 |
| AA436553 | 0,01 | -0,33 | -0,03 | 0,59 | 0,24 | 262 |
| NM_033015 | 0,00 | -0,37 | -0,06 | 0,32 | 0,25 | 263 |
| XM_002224 | 0,01 | -0,26 | 0,04 | 0,57 | 0,28 | 264 |
| AI708030 | 0,00 | 0,11 | 0,41 | 0,37 | 0,32 | 265 |
| AI041544 | 0,00 | -0,28 | 0,02 | 0,28 | 0,27 | 266 |
| NM_005801 | 0,03 | -1,39 | -1,09 | 0,36 | 0,54 | 267 |
| NM_022559 | 0,00 | -0,63 | -0,33 | 0,42 | 0,24 | 268 |
| XM_043864 | 0,00 | -0,40 | -0,10 | 0,32 | 0,31 | 269 |
| NM_003840 | 0,01 | -0,42 | -0,12 | 0,52 | 0,25 | 270 |
| AI565083 | 0,00 | -0,28 | 0,02 | 0,32 | 0,24 | 271 |
| R91168 | 0,01 | 0,15 | 0,45 | 0,36 | 0,40 | 272 |
| AI799787 | 0,01 | 0,12 | 0,41 | 0,34 | 0,38 | 273 |
| AI652564 | 0,00 | -0,91 | -0,61 | 0,31 | 0,30 | 274 |
| H05310 | 0,00 | 0,02 | 0,32 | 0,30 | 0,29 | 275 |
| AA708806 | 0,00 | -0,22 | 0,08 | 0,24 | 0,26 | 276 |
| H74205 | 0,03 | 0,07 | 0,37 | 0,42 | 0,49 | 277 |
| NM_000061 | 0,03 | -1,59 | -1,30 | 0,40 | 0,53 | 278 |
| NM_003110.3 | 0,00 | -0,29 | 0,00 | 0,38 | 0,27 | 279 |
| AA625887 | 0,00 | -0,30 | -0,01 | 0,22 | 0,23 | 280 |
| H41124 | 0,03 | -0,37 | -0,08 | 0,39 | 0,48 | 281 |
| AI769514 | 0,02 | -0,58 | -0,28 | 0,56 | 0,35 | 282 |
| XM_036107 | 0,03 | -0,27 | 0,02 | 0,27 | 0,53 | 283 |
| R52679 | 0,01 | -1,03 | -0,74 | 0,36 | 0,35 | 284 |
| Al217811 | 0,04 | 0,02 | 0,31 | 0,31 | 0,59 | 285 |
| NM_004168 | 0,02 | -0,63 | -0,34 | 0,42 | 0,44 | 286 |
| AI933607 | 0,03 | -0,24 | 0,05 | 0,32 | 0,55 | 287 |
| NM_007052.3 | 0,03 | -0,24 | 0,05 | 0,61 | 0,29 | 288 |
| AI799137 | 0,02 | -0,42 | -0,12 | 0,52 | 0,37 | 289 |
| NM_002720 | 0,00 | -0,64 | -0,35 | 0,28 | 0,34 | 290 |
| R26635 | 0,04 | -0,16 | 0,13 | 0,32 | 0,57 | 291 |
| AI625594 | 0,00 | -0,01 | 0,28 | 0,29 | 0,31 | 292 |
| NM_001562 | 0,00 | -0,42 | -0,13 | 0,22 | 0,27 | 293 |
| W93717 | 0,05 | 0,11 | 0,40 | 0,64 | 0,40 | 294 |
| NM_002521.1 | 0,01 | -0,10 | 0,19 | 0,44 | 0,29 | 295 |
| R42543 | 0,05 | 0,26 | 0,55 | 0,45 | 0,54 | 296 |
| AI302949 | 0,00 | -0,13 | 0,16 | 0,21 | 0,23 | 297 |
| H54279 | 0,00 | -0,01 | 0,27 | 0,29 | 0,32 | 298 |
| AI219513 | 0,00 | -0,47 | -0,19 | 0,41 | 0,27 | 299 |
| N68173 | 0,00 | -0,11 | 0,18 | 0,26 | 0,38 | 300 |
| AA496235 | 0,00 | -0,38 | -0,09 | 0,41 | 0,28 | 301 |
| AI742529 | 0,03 | 0,39 | 0,67 | 0,33 | 0,51 | 302 |
| H79534 | 0,01 | -0,50 | -0,21 | 0,47 | 0,32 | 303 |
| AA002267 | 0,03 | -0,04 | 0,25 | 0,36 | 0,47 | 304 |
| H52638 | 0,00 | -0,38 | -0,10 | 0,40 | 0,28 | 305 |
| N70324 | 0,01 | 0,07 | 0,35 | 0,47 | 0,33 | 306 |
| NM_003805 | 0,02 | -0,83 | -0,55 | 0,47 | 0,36 | 307 |
| N59766 | 0,03 | -0,04 | 0,24 | 0,41 | 0,43 | 308 |
| XM_034770 | 0,04 | -1,26 | -0,98 | 0,44 | 0,45 | 309 |
| AI538438 | 0,01 | 0,06 | 0,34 | 0,43 | 0,31 | 310 |
| AI250800 | 0,00 | 0,05 | 0,32 | 0,23 | 0,29 | 311 |
| AA845475 | 0,00 | -0,24 | 0,04 | 0,39 | 0,25 | 312 |
| AI700169 | 0,00 | -0,23 | 0,05 | 0,33 | 0,30 | 313 |
| NM_003639) | 0,00 | -0,37 | -0,09 | 0,26 | 0,31 | 314 |
| AI125864 | 0,03 | -0,27 | 0,01 | 0,60 | 0,24 | 315 |
| NM_000757 | 0,03 | 0,12 | 0,40 | 0,38 | 0,48 | 316 |
| NM_006216 | 0,03 | -0,12 | 0,16 | 0,45 | 0,42 | 317 |
| AI077481 | 0,04 | 0,21 | 0,49 | 0,41 | 0,48 | 318 |
| AI149647 | 0,03 | -0,13 | 0,15 | 0,43 | 0,46 | 319 |
| XM_030906 | 0,01 | 0,18 | 0,45 | 0,28 | 0,39 | 320 |
| NM 004834 | 0,00 | -0,68 | -0,41 | 0,27 | 0,35 | 321 |
| XM_031287 | 0,01 | 0,11 | 0,38 | 0,27 | 0,41 | 322 |
| AI923251 | 0,00 | -0,25 | 0,02 | 0,20 | 0,24 | 323 |
| AI203697 | 0,00 | -0,22 | 0,05 | 0,31 | 0,18 | 324 |
| AA621192 | 0,02 | 0,04 | 0,31 | 0,34 | 0,45 | 325 |
| XM_008450 | 0,02 | -0,24 | 0,03 | 0,51 | 0,29 | 326 |
| AI540674 | 0,00 | -0,76 | -0,49 | 0,25 | 0,34 | 327 |
| AA514237 | 0,03 | 0,35 | 0,62 | 0,31 | 0,47 | 328 |
| AI348271 | 0,01 | -0,11 | 0,16 | 0,45 | 0,26 | 329 |
| NM_000684.1 | 0,02 | 0,41 | 0,68 | 0,29 | 0,44 | 330 |
| NM_001951 | 0,03 | -0,67 | -0,40 | 0,50 | 0,40 | 331 |
| N55249 | 0,01 | -0,42 | -0,15 | 0,45 | 0,31 | 332 |
| AI150732 | 0,01 | -0,20 | 0,07 | 0,34 | 0,34 | 333 |
| AI147315 | 0,03 | 0,35 | 0,62 | 0,38 | 0,45 | 334 |
| NM_003010 | 0,00 | -0,46 | -0,20 | 0,34 | 0,25 | 335 |
| AA460460 | 0,01 | -0,22 | 0,05 | 0,47 | 0,28 | 336 |
| AI651337 | 0,01 | -0,49 | -0,22 | 0,41 | 0,31 | 337 |
| AA971087 | 0,01 | -0,19 | 0,08 | 0,42 | 0,25 | 338 |
| NM_003811 | 0,03 | -1,09 | -0,82 | 0,50 | 0,37 | 339 |
| XM_053519 | 0,01 | -0,30 | -0,04 | 0,26 | 0,39 | 340 |
| NM_001609.1 | 0,00 | -0,24 | 0,03 | 0,29 | 0,29 | 341 |
| AA463423 | 0,00 | -0,17 | 0,09 | 0,22 | 0,35 | 342 |
| AA648848 | 0,02 | 0,04 | 0,30 | 0,35 | 0,42 | 343 |
| AI141692 | 0,05 | -0,12 | 0,14 | 0,67 | 0,22 | 344 |
| R79239 | 0,04 | 0,06 | 0,33 | 0,50 | 0,42 | 345 |
| AI298171 | 0,00 | -0,28 | -0,01 | 0,17 | 0,21 | 346 |
| H17432 | 0,03 | -0,29 | -0,03 | 0,57 | 0,23 | 347 |
| NM_004635 | 0,05 | -1,36 | -1,10 | 0,31 | 0,51 | 348 |
| NM_005409 | 0,02 | 0,14 | 0,40 | 0,20 | 0,50 | 349 |
| AI452845 | 0,03 | 0,23 | 0,49 | 0,37 | 0,43 | 350 |
| AI222914 | 0,00 | -0,03 | 0,23 | 0,29 | 0,24 | 351 |
| AI885492 | 0,00 | -0,06 | 0,20 | 0,34 | 0,20 | 352 |
| NM 002953 | 0,01 | -0,61 | -0,35 | 0,24 | 0,41 | 353 |
| AI201175 | 0,01 | 0,25 | 0,51 | 0,30 | 0,33 | 354 |
| NM 001735 | 0,02 | -0,45 | -0,20 | 0,47 | 0,29 | 355 |
| D78151 | 0,02 | -0,70 | -0,44 | 0,42 | 0,34 | 356 |
| NM 006712 | 0,00 | -0,20 | 0,06 | 0,36 | 0,24 | 357 |
| AF004429 | 0,00 | -0,63 | -0,37 | 0,29 | 0,29 | 358 |
| NM_031409 | 0,03 | 0,19 | 0,44 | 0,32 | 0,45 | 359 |
| AI742287 | 0,01 | -0,30 | -0,04 | 0,41 | 0,27 | 360 |
| BC015542 | 0,02 | -0,42 | -0,16 | 0,34 | 0,42 | 361 |
| AI685923 | 0,00 | -0,43 | -0,18 | 0,32 | 0,22 | 362 |
| NM 002218.1 | 0,01 | -0,55 | -0,29 | 0,34 | 0,28 | 363 |
| XM_003913 | 0,00 | -0,05 | 0,20 | 0,29 | 0,29 | 364 |
| N53480 | 0,02 | -0,64 | -0,39 | 0,40 | 0,38 | 365 |
| XM_048511 | 0,00 | -0,35 | -0,10 | 0,37 | 0,25 | 366 |
| R06710 | 0,02 | 0,05 | 0,30 | 0,39 | 0,35 | 367 |
| AI694720 | 0,01 | 0,29 | 0,54 | 0,28 | 0,34 | 368 |
| AI910988 | 0,00 | 0,07 | 0,32 | 0,23 | 0,33 | 369 |
| AA411624 | 0,02 | -0,52 | -0,27 | 0,33 | 0,38 | 370 |
| BC024270 | 0,00 | -0,43 | -0,18 | 0,32 | 0,27 | 371 |
| T90460 | 0,01 | -0,39 | -0,14 | 0,48 | 0,17 | 372 |
| NM 004850 | 0,02 | -0,92 | -0,67 | 0,41 | 0,32 | 373 |
| AA044390 | 0,03 | -0,01 | 0.24 | 0,26 | 0,45 | 374 |
| NM 005347.2 | 0,00 | -0,26 | -0,01 | 0,21 | 0,23 | 375 |
| XM_027216 | 0,03 | -0,68 | -0,43 | 0,42 | 0,39 | 376 |
| H53259 | 0,04 | 0,45 | 0,70 | 0,35 | 0,45 | 377 |
| R26717 | 0,02 | -0,02 | 0,22 | 0,40 | 0,35 | 378 |
| AI912970 | 0,02 | 0,19 | 0,44 | 0,36 | 0,35 | 379 |
| XM_001687 | 0,04 | -0,91 | -0,66 | 0,44 | 0,40 | 380 |
| NM_000565 | 0,04 | -0,67 | -0,43 | 0,27 | 0,50 | 381 |
| AI374990 | 0,01 | -0,14 | 0,10 | 0,31 | 0,35 | 382 |
| N22563 | 0,02 | 0,12 | 0,37 | 0,33 | 0,39 | 383 |
| AI764969 | 0,03 | -0,18 | 0,07 | 0,53 | 0,25 | 384 |
| AA417950 | 0,02 | -0,34 | -0,09 | 0,48 | 0,26 | 385 |
| H15431 | 0,03 | -0,39 | -0,14 | 0,51 | 0,30 | 386 |
| AI147997 | 0,02 | 0,11 | 0,35 | 0,24 | 0,45 | 387 |
| AI378142 | 0,03 | -0,10 | 0,14 | 0,25 | 0,42 | 388 |
| AA528101 | 0,00 | -0,43 | -0,19 | 0,32 | 0,21 | 389 |
| T83761 | 0,04 | -0,41 | -0,16 | 0,36 | 0,46 | 390 |
| XM 046674 | 0,04 | -0,80 | -0,56 | 0,59 | 0,21 | 391 |
| AI925556 | 0,00 | -0,53 | -0,29 | 0,24 | 0,21 | 392 |
| N50785 | 0,03 | 0,25 | 0,49 | 0,29 | 0,43 | 393 |
| AI739085 | 0,04 | -0,41 | -0,17 | 0,41 | 0,39 | 394 |
| AA885052 | 0,02 | -0,13 | 0,11 | 0,42 | 0,28 | 395 |
| R45218 | 0,01 | 0,17 | 0,41 | 0,25 | 0,37 | 396 |
| N71365 | 0,00 | -0,39 | -0,15 | 0,22 | 0,33 | 397 |
| AI590053 | 0,00 | -0,38 | -0,14 | 0,19 | 0,16 | 398 |
| NM 013229 | 0,00 | 0,02 | 0,26 | 0,22 | 0,26 | 399 |
| NM_001196 | 0,02 | -0,53 | -0,30 | 0,27 | 0,39 | 400 |
| R94509 | 0,01 | 0,07 | 0,31 | 0,29 | 0,29 | 401 |
| AA282936 | 0,02 | 0,16 | 0,39 | 0,41 | 0,33 | 402 |
| NM 003824 | 0,01 | -0,74 | -0,50 | 0,26 | 0,37 | 403 |
| T65296 | 0,01 | 0,00 | 0,23 | 0,23 | 0,34 | 404 |
| AI583064 | 0,03 | 0,01 | 0,24 | 0,28 | 0,42 | 405 |
| R94626 | 0,01 | -0,25 | -0,01 | 0,25 | 0,35 | 406 |
| AI216612 | 0,03 | -0,20 | 0,03 | 0,42 | 0,35 | 407 |
| NM 015318.1 | 0,01 | -0,12 | 0,11 | 0,32 | 0,25 | 408 |
| AA426397 | 0,02 | -0,37 | -0,14 | 0,33 | 0,34 | 409 |
| H78362 | 0,01 | -0,53 | -0,30 | 0,31 | 0,27 | 410 |
| AA878269 | 0,02 | -0,28 | -0,05 | 0,32 | 0,34 | 411 |
| NM_017778 | 0,01 | -0,17 | 0,06 | 0,39 | 0,26 | 412 |
| AI709236 | 0,00 | -0,24 | -0,01 | 0,21 | 0,28 | 413 |
| AA465175 | 0,01 | -0,11 | 0,12 | 0,32 | 0,27 | 414 |
| AI798573 | 0,00 | -0,34 | -0,12 | 0,18 | 0,21 | 415 |
| NM_139070 | 0,02 | -0,87 | -0,64 | 0,41 | 0,27 | 416 |
| XM_049749 | 0,04 | -0,18 | 0,05 | 0,50 | 0,28 | 417 |
| AI864931 | 0,01 | 0,09 | 0,31 | 0,24 | 0,31 | 418 |
| NM 021975 | 0,00 | -0,63 | -0,41 | 0,29 | 0,22 | 419 |
| R60931 | 0,03 | 0,16 | 0,39 | 0,35 | 0,35 | 420 |
| XM_037260 | 0,00 | -0,38 | -0,15 | 0,18 | 0,26 | 421 |
| R36650 | 0,00 | -0,40 | -0,17 | 0,23 | 0,25 | 422 |
| AA621075 | 0,00 | -0,31 | -0,09 | 0,24 | 0,28 | 423 |
| AI018273 | 0,04 | -0,23 | 0,00 | 0,45 | 0,31 | 424 |
| AI701905 | 0,00 | -0,24 | -0,01 | 0,22 | 0,24 | 425 |
| XM_054686 | 0,02 | -0,55 | -0,33 | 0,30 | 0,31 | 426 |
| NM_139276 | 0,02 | -0,09 | 0,14 | 0,25 | 0,38 | 427 |
| AI418064 | 0,01 | -0,23 | -0,01 | 0,32 | 0,24 | 428 |
| NM_002503 | 0,03 | -0,50 | -0,28 | 0,40 | 0,32 | 429 |
| AI923559 | 0,02 | -0,37 | -0,15 | 0,35 | 0,33 | 430 |
| NM_004295 | 0,04 | -0,66 | -0,44 | 0,40 | 0,37 | 431 |
| AA425105 | 0,03 | -0,19 | 0,04 | 0,43 | 0,29 | 432 |
| NM_002997 | 0,02 | 0,01 | 0,24 | 0,37 | 0,28 | 433 |
| NM 024013 | 0,00 | 0,04 | 0,27 | 0,28 | 0,23 | 434 |
| AA856755 | 0,02 | -0,40 | -0,18 | 0,34 | 0,34 | 435 |
| AI371339 | 0,00 | -0,28 | -0,06 | 0,24 | 0,22 | 436 |
| AA453528 | 0,04 | -0,30 | -0,08 | 0,50 | 0,27 | 437 |
| AI214646 | 0,04 | -0,27 | -0,05 | 0,24 | 0,43 | 438 |
| NM_006724 | 0,01 | -0,45 | -0,22 | 0,32 | 0,24 | 439 |
| AI925740 | 0,02 | 0,02 | 0,24 | 0,42 | 0,25 | 440 |
| H81378 | 0,00 | -0,16 | 0,06 | 0,32 | 0,19 | 441 |
| H82860 | 0,03 | 0,01 | 0,23 | 0,25 | 0,41 | 442 |
| BC032713 | 0,02 | 0,39 | 0,61 | 0,22 | 0,37 | 443 |
| H10036 | 0,05 | -0,08 | 0,13 | 0,42 | 0,35 | 444 |
| AI707917 | 0,00 | -0,34 | -0,12 | 0,18 | 0,20 | 445 |
| AA676928 | 0,05 | -0,04 | 0,18 | 0,36 | 0,40 | 446 |
| AI057616 | 0,00 | -0,03 | 0,19 | 0,19 | 0,30 | 447 |
| NM_03080 | 0,01 | -0,03 | 0,18 | 0,29 | 0,25 | 448 |
| AI685198 | 0,00 | -0,41 | -0,20 | 0,19 | 0,23 | 449 |
| AA436683 | 0,02 | -0,16 | 0,05 | 0,34 | 0,29 | 450 |
| R39456 | 0,00 | -0,34 | -0,13 | 0,17 | 0,23 | 451 |
| NM_004050 | 0,03 | -0,23 | -0,02 | 0,38 | 0,31 | 452 |
| N49208 | 0,02 | 0,13 | 0,34 | 0,35 | 0,32 | 453 |
| XM_055699 | 0,05 | 0,02 | 0,23 | 0,36 | 0,39 | 454 |
| BC028234 | 0,02 | -0,45 | -0,24 | 0,26 | 0,37 | 455 |
| N89900 | 0,02 | -0,39 | -0,18 | 0,36 | 0,28 | 456 |
| NM_001278 | 0,00 | -0,63 | -0,42 | 0,22 | 0,21 | 457 |
| AI921613 | 0,01 | -0,06 | 0,16 | 0,25 | 0,26 | 458 |
| NM_003821 | 0,03 | -0,64 | -0,43 | 0,43 | 0,23 | 459 |
| XM_046035 | 0,00 | -0,37 | -0,16 | 0,20 | 0,27 | 460 |
| AI936300 | 0,04 | 0,08 | 0,29 | 0,30 | 0,39 | 461 |
| NM_003131 | 0,00 | -0,71 | -0,50 | 0,30 | 0,21 | 462 |
| R61546 | 0,01 | -0,52 | -0,31 | 0,30 | 0,25 | 463 |
| AA431750 | 0,02 | -0,24 | -0,03 | 0,32 | 0,29 | 464 |
| AI524099 | 0,00 | -0,03 | 0,18 | 0,15 | 0,20 | 465 |
| XM_042665 | 0,00 | 0,07 | 0,28 | 0,25 | 0,22 | 466 |
| AI820873 | 0,02 | -0,48 | -0,27 | 0,39 | 0,24 | 467 |
| NM_019011 | 0,02 | -0,60 | -0,39 | 0,27 | 0,35 | 468 |
| H51585 | 0,05 | -0,57 | -0,36 | 0,42 | 0,30 | 469 |
| AI393173 | 0,02 | -0,04 | 0,16 | 0,25 | 0,34 | 470 |
| AI560205 | 0,00 | -0,36 | -0,15 | 0,19 | 0,23 | 471 |
| AA429020 | 0,00 | -0,31 | -0,11 | 0,20 | 0,17 | 472 |
| NM_000681.2 | 0,01 | 0,14 | 0,34 | 0,30 | 0,27 | 473 |
| NM_014550 | 0,00 | -0,10 | 0,10 | 0,25 | 0,16 | 474 |
| AA453256 | 0,00 | -0,04 | 0,16 | 0,20 | 0,26 | 475 |
| NM_021138 | 0,00 | -0,23 | -0,03 | 0,28 | 0,14 | 476 |
| R51304 | 0,05 | -0,02 | 0,19 | 0,32 | 0,38 | 477 |
| AI590111 | 0,00 | -0,31 | -0,10 | 0,14 | 0,20 | 478 |
| H09305 | 0,01 | -0,57 | -0,37 | 0,31 | 0,26 | 479 |
| R99076 | 0,00 | -0,40 | -0,19 | 0,19 | 0,22 | 480 |
| AI559096 | 0,01 | 0,28 | 0,48 | 0,29 | 0,29 | 481 |
| AI610213 | 0,02 | -0,12 | 0,08 | 0,34 | 0,27 | 482 |
| N66038 | 0,00 | -0,33 | -0,12 | 0,17 | 0,20 | 483 |
| NM_002649 | 0,00 | -0,33 | -0,13 | 0,17 | 0,29 | 484 |
| NM_006676 | 0,02 | -0,54 | -0,34 | 0,32 | 0,27 | 485 |
| NM_014959 | 0,00 | -0,38 | -0,18 | 0,24 | 0,25 | 486 |
| BC013992 | 0,01 | 0,01 | 0,21 | 0,16 | 0,32 | 487 |
| N32057 | 0,02 | -0,34 | -0,14 | 0,39 | 0,20 | 488 |
| AI801695 | 0,00 | -0,33 | -0,13 | 0,17 | 0,18 | 489 |
| AI568793 | 0,03 | 0,11 | 0,31 | 0,29 | 0,33 | 490 |
| AA479285 | 0,00 | 0,08 | 0,27 | 0,23 | 0,23 | 491 |
| H06501 | 0,02 | -0,10 | 0,10 | 0,32 | 0,28 | 492 |
| R00259 | 0,03 | -0,06 | 0,14 | 0,31 | 0,32 | 493 |
| AI362368 | 0,00 | -0,33 | -0,13 | 0,17 | 0,19 | 494 |
| AI635040 | 0,00 | -0,14 | 0,06 | 0,18 | 0,26 | 495 |
| AI354869 | 0,03 | -0,48 | -0,29 | 0,32 | 0,26 | 496 |
| N71407 | 0,02 | 0,06 | 0,25 | 0,26 | 0,32 | 497 |
| XM_038544 | 0,04 | -0,12 | 0,07 | 0,38 | 0,29 | 498 |
| NM_031910 | 0,04 | 0,18 | 0,38 | 0,27 | 0,38 | 499 |
| AI862063 | 0,00 | -0,28 | -0,08 | 0,20 | 0,24 | 500 |
| AA455638 | 0,03 | 0,07 | 0,27 | 0,28 | 0,32 | 501 |
| AI697430 | 0,00 | -0,36 | -0,17 | 0,18 | 0,21 | 502 |
| R42480 | 0,01 | -0,49 | -0,29 | 0,25 | 0,23 | 503 |
| AI674115 | 0,01 | 0,02 | 0,21 | 0,24 | 0,29 | 504 |
| AA968926 | 0,03 | -0,32 | -0,13 | 0,37 | 0,26 | 505 |
| AI524694 | 0,00 | -0,38 | -0,19 | 0,18 | 0,23 | 506 |
| AA609857 | 0,02 | -0,08 | 0,12 | 0,30 | 0,29 | 507 |
| AI913713 | 0,01 | -0,46 | -0,27 | 0,33 | 0,21 | 508 |
| WO4695 | 0,00 | -0,28 | -0,09 | 0,16 | 0,24 | 509 |
| NM_033012 | 0,04 | -0,12 | 0,07 | 0,35 | 0,31 | 510 |
| T77048 | 0,02 | -0,01 | 0,18 | 0,26 | 0,31 | 511 |
| AI817381 | 0,01 | -0,25 | -0,06 | 0,23 | 0,24 | 512 |
| AI624918 | 0,03 | -0,02 | 0,17 | 0,28 | 0,32 | 513 |
| AI888072 | 0,01 | -0,26 | -0,07 | 0,23 | 0,26 | 514 |
| AA883759 | 0,00 | -0,38 | -0,20 | 0,21 | 0,22 | 515 |
| AA478611 | 0,00 | -0,34 | -0,15 | 0,25 | 0,17 | 516 |
| AI452862 | 0,03 | -0,28 | -0,09 | 0,34 | 0,26 | 517 |
| AI277855 | 0,00 | -0,46 | -0,27 | 0,24 | 0,22 | 518 |
| AI520967 | 0,00 | -0,34 | -0,15 | 0,17 | 0,20 | 519 |
| T91937 | 0,05 | 0,36 | 0,54 | 0,27 | 0,37 | 520 |
| AA993698 | 0,00 | 0,01 | 0,20 | 0,21 | 0,20 | 521 |
| AI620374 | 0,00 | -0,40 | -0,22 | 0,18 | 0,22 | 522 |
| AA707628 | 0,00 | -0,27 | -0,08 | 0,12 | 0,17 | 523 |
| AI572545 | 0,01 | -0,38 | -0,19 | 0,17 | 0,28 | 524 |
| AI801540 | 0,04 | -0,16 | 0,03 | 0,36 | 0,28 | 525 |
| AI354889 | 0,00 | -0,11 | 0,07 | 0,22 | 0,18 | 526 |
| NM_030751 | 0,03 | -0,09 | 0,09 | 0,33 | 0,25 | 527 |
| NM_000657 | 0,01 | -0,50 | -0,32 | 0,27 | 0,22 | 528 |
| AA045139 | 0,02 | -0,43 | -0,24 | 0,34 | 0,23 | 529 |
| AI912148 | 0,00 | -0,25 | -0,06 | 0,18 | 0,20 | 530 |
| AA513806 | 0,04 | -0,21 | -0,03 | 0,29 | 0,32 | 531 |
| H48440 | 0,00 | -0,35 | -0,17 | 0,16 | 0,23 | 532 |
| AA114117 | 0,00 | -0,38 | -0,20 | 0,17 | 0,18 | 533 |
| AI654471 | 0,00 | -0,20 | -0.02 | 0,19 | 0,20 | 534 |
| AA423792 | 0,00 | -0,16 | 0,02 | 0,14 | 0,25 | 535 |
| AI926484 | 0,00 | -0,08 | 0,10 | 0,25 | 0,14 | 536 |
| T89979 | 0,00 | -0,30 | -0,12 | 0,16 | 0,19 | 537 |
| AI889310 | 0,01 | -0,25 | -0,07 | 0,26 | 0,21 | 538 |
| R11261 | 0,04 | -0,27 | -0,09 | 0,43 | 0,18 | 539 |
| AI932551 | 0,00 | -0,32 | -0,14 | 0,16 | 0,23 | 540 |
| NM_017626.1 | 0,01 | -0,56 | -0,38 | 0,22 | 0,26 | 541 |
| AI381513 | 0,04 | -0,29 | -0,11 | 0,32 | 0,29 | 542 |
| AA682407 | 0,02 | -0,24 | -0,07 | 0,25 | 0,29 | 543 |
| AA954316 | 0,04 | -0,75 | -0,57 | 0,35 | 0,27 | 544 |
| AI791500 | 0,02 | 0,16 | 0,34 | 0,18 | 0,31 | 545 |
| T91881 | 0,00 | -0,26 | -0,08 | 0,18 | 0,23 | 546 |
| AI149857 | 0,02 | -0,06 | 0,12 | 0,18 | 0,30 | 547 |
| AI370842 | 0,04 | 0,11 | 0,29 | 0,31 | 0,30 | 548 |
| AA401205 | 0,00 | -0,13 | 0,05 | 0,18 | 0,19 | 549 |
| AA453267 | 0,02 | -0,03 | 0,15 | 0,26 | 0,28 | 550 |
| R88475 | 0,00 | -0,35 | -0,17 | 0,18 | 0,20 | 551 |
| AI864919 | 0,01 | -0,38 | -0,20 | 0,19 | 0,25 | 552 |
| NM_002169 | 0,04 | -0,24 | -0,07 | 0,33 | 0,27 | 553 |
| R46801 | 0,05 | 0,27 | 0,44 | 0,35 | 0,27 | 554 |
| AI277856 | 0,02 | -0,12 | 0,06 | 0,22 | 0,27 | 555 |
| H22921 | 0,00 | -0,33 | -0,15 | 0,19 | 0,22 | 556 |
| AI763386 | 0,03 | -0,37 | -0,20 | 0,30 | 0,27 | 557 |
| N78812 | 0,01 | -0,23 | -0,06 | 0,25 | 0,20 | 558 |
| H83981 | 0,04 | 0,04 | 0,22 | 0,28 | 0,30 | 559 |
| AA029887 | 0,00 | -0,40 | -0,22 | 0,19 | 0,21 | 560 |
| AI192112 | 0,00 | -0,11 | 0,06 | 0,15 | 0,24 | 561 |
| W88960 | 0,01 | 0,10 | 0,28 | 0,21 | 0,24 | 562 |
| W80744 | 0,00 | -0,25 | -0,08 | 0,15 | 0,21 | 563 |
| AI521577 | 0,01 | -0,31 | -0,13 | 0,18 | 0,23 | 564 |
| AA418572 | 0,01 | -0,13 | 0,05 | 0,17 | 0,25 | 565 |
| N73510 | 0,00 | -0,38 | -0,21 | 0,17 | 0,22 | 566 |
| AI631299 | 0,03 | -0,16 | 0,01 | 0,24 | 0,29 | 567 |
| XM_012717 | 0,00 | -0,44 | -0,27 | 0,18 | 0,17 | 568 |
| NM_000590 | 0,03 | 0,32 | 0,50 | 0,23 | 0,29 | 569 |
| AI381910 | 0,01 | -0,04 | 0,13 | 0,21 | 0,23 | 570 |
| R87714 | 0,04 | -0,18 | -0,01 | 0,33 | 0,23 | 571 |
| AA609628 | 0,00 | -0,36 | -0,19 | 0,17 | 0,19 | 572 |
| AA634317 | 0,03 | 0,19 | 0,36 | 0,27 | 0,28 | 573 |
| AI214830 | 0,04 | -0,27 | -0,10 | 0,23 | 0,32 | 574 |
| AI203201 | 0,04 | -0,26 | -0,09 | 0,26 | 0,29 | 575 |
| AI924806 | 0,00 | -0,29 | -0,12 | 0,20 | 0,18 | 576 |
| AA701319 | 0,02 | -0,07 | 0,10 | 0,22 | 0,28 | 577 |
| N63628 | 0,03 | -0,26 | -0,09 | 0,26 | 0,27 | 578 |
| R02742 | 0,04 | -0,17 | -0,01 | 0,32 | 0,25 | 579 |
| H07860 | 0,02 | -0,03 | 0,13 | 0,26 | 0,24 | 580 |
| H77534 | 0,02 | -0,35 | -0,18 | 0,32 | 0,20 | 581 |
| AI208537 | 0,02 | -0,21 | -0,04 | 0,34 | 0,17 | 582 |
| AI184715 | 0,01 | -0,03 | 0,13 | 0,23 | 0,20 | 583 |
| R05816 | 0,00 | -0,27 | -0,10 | 0,19 | 0,20 | 584 |
| AA961252 | 0,04 | -0,14 | 0,02 | 0,26 | 0,31 | 585 |
| AI801425 | 0,00 | -0,21 | -0,04 | 0,22 | 0,17 | 586 |
| AA477776 | 0,01 | -0,01 | 0,16 | 0,21 | 0,20 | 587 |
| R06585 | 0,01 | -0,40 | -0,23 | 0,18 | 0,21 | 588 |
| AA405788 | 0.01 | -0,36 | -0,19 | 0,15 | 0,25 | 589 |
| R06107 | 0,01 | -0,23 | -0,07 | 0,24 | 0,19 | 590 |
| AA923316 | 0,00 | -0,20 | -0,04 | 0,15 | 0,19 | 591 |
| AI421397 | 0,02 | -0,02 | 0,14 | 0,19 | 0,26 | 592 |
| NM_006881 | 0,01 | -0,40 | -0,24 | 0,20 | 0,23 | 593 |
| R43415 | 0,00 | -0,24 | -0,08 | 0,14 | 0,19 | 594 |
| H11495 | 0,01 | -0,29 | -0,12 | 0,26 | 0,13 | 595 |
| AI208772 | 0,04 | -0,23 | -0,07 | 0,29 | 0,27 | 596 |
| AA479784 | 0,03 | -0,06 | 0,10 | 0,29 | 0,24 | 597 |
| AA485092 | 0,00 | -0,36 | -0,20 | 0,16 | 0,20 | 598 |
| AA664688 | 0,00 | -0,39 | -0,23 | 0,18 | 0,20 | 599 |
| H48230 | 0,01 | -0,29 | -0,13 | 0,19 | 0,21 | 600 |
| AI248075 | 0,02 | -0,12 | 0,04 | 0,25 | 0,22 | 601 |
| AA418695 | 0,04 | -0,01 | 0,15 | 0,21 | 0,31 | 602 |
| AI673731 | 0,01 | -0,41 | -0,25 | 0,16 | 0,22 | 603 |
| XM_008948 | 0,03 | 0,10 | 0,26 | 0,26 | 0,26 | 604 |
| AI301257 | 0,00 | -0,31 | -0,15 | 0,19 | 0,19 | 605 |
| NM_003823 | 0,04 | -0,72 | -0,56 | 0,31 | 0,24 | 606 |
| AI744264 | 0,01 | -0,14 | 0,02 | 0,16 | 0,22 | 607 |
| AI809873 | 0,03 | -0,45 | -0,29 | 0,24 | 0,26 | 608 |
| AI354243 | 0,01 | -0,34 | -0,18 | 0,17 | 0,21 | 609 |
| NM_001553.1 | 0,04 | -0,15 | 0,01 | 0,27 | 0.27 | 610 |
| W86575 | 0,02 | -0,34 | -0,18 | 0,23 | 0,24 | 611 |
| AA442720 | 0,03 | -0,15 | 0,01 | 0,27 | 0,24 | 612 |
| AA993597 | 0,03 | 0,17 | 0,33 | 0,26 | 0,24 | 613 |
| A1433952 | 0,01 | -0,30 | -0,14 | 0,16 | 0,23 | 614 |
| R56800 | 0,01 | -0,09 | 0,06 | 0,17 | 0,21 | 615 |
| AA417031 | 0,01 | -0,21 | -0,06 | 0,19 | 0,23 | 616 |
| R53961 | 0,04 | -0,45 | -0,29 | 0,28 | 0,25 | 617 |
| T86887 | 0,00 | -0,23 | -0,08 | 0,13 | 0,20 | 618 |
| AA705808 | 0,01 | -0,20 | -0,04 | 0,25 | 0,18 | 619 |
| AA426451 | 0,00 | -0,28 | -0,13 | 0,16 | 0,19 | 620 |
| H06263 | 0,00 | -0,28 | -0,12 | 0,14 | 0,17 | 621 |
| AA659421 | 0,00 | -0,32 | -0,17 | 0,14 | 0,16 | 622 |
| AI801595 | 0,00 | -0,28 | -0,13 | 0,16 | 0,19 | 623 |
| AI672318 | 0,04 | -0,20 | -0,05 | 0,31 | 0,24 | 624 |
| AI762019 | 0.01 | -0,25 | -0,09 | 0.19 | 0,21 | 625 |
| N92873 | 0,02 | -0,11 | 0,05 | 0,28 | 0,19 | 626 |
| NM_017442 | 0,04 | 0,08 | 0,23 | 0,28 | 0,25 | 627 |
| H46164 | 0,03 | 0,03 | 0,18 | 0,21 | 0,27 | 628 |
| T83946 | 0,01 | -0,29 | -0,14 | 0,20 | 0,21 | 629 |
| AA868726 | 0,04 | -0,42 | -0,27 | 0,26 | 0,25 | 630 |
| H88129 | 0,02 | -0,37 | -0,22 | 0,21 | 0,23 | 631 |
| R88267 | 0,04 | -0,12 | 0,03 | 0,30 | 0,23 | 632 |
| AI798545 | 0,01 | -0,32 | -0,17 | 0,17 | 0.19 | 633 |
| N57775 | 0,02 | -0,14 | 0,01 | 0,22 | 0,22 | 634 |
| AA425134 | 0,00 | -0,21 | -0,07 | 0,16 | 0,19 | 635 |
| AI744807 | 0,01 | -0,59 | -0.44 | 0,20 | 0,22 | 636 |
| AI702056 | 0,05 | -0,27 | -0,12 | 0,22 | 0,29 | 637 |
| NM_000575 | 0,04 | -0,27 | -0,12 | 0,23 | 0,25 | 638 |
| T98779 | 0,01 | -0,38 | -0,23 | 0,18 | 0,23 | 639 |
| NM_000587 | 0,01 | -0,43 | -0,28 | 0,18 | 0.19 | 640 |
| R92455 | 0,01 | -0.36 | -0,21 | 0,17 | 0,21 | 641 |
| AI758473 | 0,01 | -0,36 | -0,21 | 0,18 | 0,22 | 642 |
| AA398364 | 0,00 | -0,31 | -0,17 | 0,13 | 0,21 | 643 |
| AI811774 | 0,05 | 0,20 | 0,35 | 0,23 | 0,27 | 644 |
| AI299411 | 0,00 | -0,24 | -0,10 | 0,17 | 0,18 | 645 |
| AA225138 | 0,00 | -0,26 | -0,11 | 0,12 | 0,17 | 646 |
| AA418689 | 0,05 | 0,09 | 0,24 | 0,21 | 0,28 | 647 |
| T77995 | 0,01 | -0,18 | -0,04 | 0,21 | 0,20 | 648 |
| AA808788 | 0,04 | -0,33 | -0,18 | 0,18 | 0,25 | 649 |
| AI677645 | 0,01 | -0,25 | -0,11 | 0,15 | 0,19 | 650 |
| AA629306 | 0,04 | -0,07 | 0,07 | 0,26 | 0,24 | 651 |
| AA749151 | 0.00 | -0,19 | -0,05 | 0,13 | 0,17 | 652 |
| AI679294 | 0,01 | -0,41 | -0,27 | 0.19 | 0,19 | 653 |
| R45611 | 0,02 | -0,24 | -0,10 | 0.16 | 0,24 | 654 |
| NM_000588 | 0,05 | -0,18 | -0,04 | 0,29 | 0,22 | 655 |
| H99483 | 0,01 | -0,29 | -0,15 | 0,16 | 0,22 | 656 |
| AI679923 | 0,01 | -0,46 | -0,32 | 0,17 | 0,20 | 657 |
| AI077580 | 0,05 | -0,04 | 0,10 | 0,27 | 0,23 | 658 |
| D49410 | 0,01 | -0.31 | -0,17 | 0,19 | 0,19 | 659 |
| AI692267 | 0,04 | -0,42 | -0,28 | 0,22 | 0,24 | 660 |
| AI804001 | 0,02 | 0,00 | 0,14 | 0,19 | 0,23 | 661 |
| T87188 | 0,01 | -0,32 | -0,18 | 0,19 | 0,19 | 662 |
| AI368218 | 0,02 | -0,24 | -0.10 | 0,13 | 0,23 | 663 |
| AI208749 | 0,02 | -0,02 | 0,11 | 0,22 | 0,19 | 664 |
| H61046 | 0,02 | -0.21 | -0,07 | 0,18 | 0,22 | 665 |
| NM_001330.1 | 0,01 | -0,05 | 0,08 | 0,19 | 0,20 | 666 |
| XM_001322 | 0,01 | -0,33 | -0,19 | 0,18 | 0,17 | 667 |
| NM_004195 | 0,04 | 0,23 | 0,37 | 0,16 | 0,27 | 668 |
| AI285713 | 0,01 | -0,32 | -0,18 | 0,15 | 0,21 | 669 |
| AA527369 | 0,00 | -0,13 | 0,00 | 0,15 | 0,16 | 670 |
| AI350069 | 0,01 | -0,24 | -0,11 | 0,15 | 0,21 | 671 |
| AI493975 | 0,01 | -0,24 | -0,10 | 0,18 | 0,16 | 672 |
| AI355007 | 0,03 | -0,23 | -0,10 | 0,22 | 0.21 | 673 |
| AA225239 | 0,04 | -0,40 | -0,26 | 0,21 | 0,25 | 674 |
| AA001392 | 0,03 | -0,39 | -0,26 | 0,24 | 0.19 | 675 |
| AI933797 | 0,02 | -0,28 | -0,15 | 0,22 | 0,18 | 676 |
| R43065 | 0,01 | -0,21 | -0,08 | 0,16 | 0,18 | 677 |
| AA478621 | 0,03 | -0,21 | -0,08 | 0,21 | 0,20 | 678 |
| AA012850 | 0,03 | -0,32 | -0,19 | 0,17 | 0,22 | 679 |
| AI925035 | 0,03 | -0,15 | -0,02 | 0,17 | 0,23 | 680 |
| AA995218 | 0,03 | -0,22 | -0,09 | 0.19 | 0,21 | 681 |
| AA897716 | 0,04 | -0,18 | -0,06 | 0,23 | 0,21 | 682 |
| AA983987 | 0,02 | -0,28 | -0,15 | 0,18 | 0,18 | 683 |
| AI762202 | 0,03 | -0,18 | -0,05 | 0,22 | 0,20 | 684 |
| T95909 | 0,02 | -0,34 | -0,22 | 0,18 | 0,19 | 685 |
| N22551 | 0,03 | -0,36 | -0,24 | 0,17 | 0,22 | 686 |
| AI769053 | 0,03 | -0,28 | -0,15 | 0,15 | 0,22 | 687 |
| AF039955 | 0,01 | -0,37 | -0,24 | 0,20 | 0,16 | 688 |
| AI935874 | 0,02 | -0,26 | -0,14 | 0,16 | 0.20 | 689 |
| AI570779 | 0,01 | -0,31 | -0,19 | 0,15 | 0,18 | 690 |
| AI240539 | 0,01 | -0,22 | -0,09 | 0,17 | 0,18 | 691 |
| H54423 | 0,03 | -0,32 | -0,20 | 0,16 | 0,22 | 692 |
| AA460136 | 0,02 | -0,09 | 0,03 | 0,24 | 0,14 | 693 |
| NM_033357 | 0,05 | -0,24 | -0,12 | 0,19 | 0,23 | 694 |
| AI923479 | 0.04 | -0,30 | -0,18 | 0.20 | 0,22 | 695 |
| H18944 | 0,04 | -0,42 | -0,30 | 0,21 | 0,19 | 696 |
| NM_006509 | 0,03 | 0,01 | 0,13 | 0,12 | 0,23 | 697 |
| AI865298 | 0,02 | -0,31 | -0,19 | 0,13 | 0,20 | 698 |
| A1123502 | 0,04 | -0,36 | -0,24 | 0,17 | 0,22 | 699 |
| AI885918 | 0,02 | -0,24 | -0,12 | 0,14 | 0.19 | 700 |
| AA225023 | 0,02 | -0,33 | -0,21 | 0,12 | 0,20 | 701 |
| AA421020 | 0,04 | -0,25 | -0,13 | 0,19 | 0,21 | 702 |
| AJ297560 | 0,05 | -0,29 | -0,17 | 0,21 | 0,20 | 703 |
| N95217 | 0,02 | -0,30 | -0,19 | 0,12 | 0,19 | 704 |
| AA526032 | 0,04 | -0,25 | -0,13 | 0,20 | 0,19 | 705 |
| AA496309 | 0,02 | -0,32 | -0,20 | 0,15 | 0,19 | 706 |
| AI732958 | 0,03 | -0,22 | -0,11 | 0,18 | 0,18 | 707 |
| AA410828 | 0,02 | -0,29 | -0,18 | 0,20 | 0,16 | 708 |
| AA453993 | 0,02 | -0,30 | -0,19 | 0,18 | 0,16 | 709 |
| R92993 | 0,02 | -0,26 | -0,15 | 0,12 | 0,19 | 710 |
| NM_003921 | 0,04 | -0,23 | -0,13 | 0.20 | 0,18 | 711 |
| AI379967 | 0,02 | -0,34 | -0,23 | 0,15 | 0,17 | 712 |
| AI926656 | 0,04 | -0,26 | -0,15 | 0,18 | 0,19 | 713 |
| AA935872 | 0,03 | -0,31 | -0,20 | 0,16 | 0,18 | 714 |
| H08791 | 0,03 | -0,27 | -0,17 | 0,16 | 0,18 | 715 |
| AI932884 | 0,03 | -0.31 | -0,21 | 0,16 | 0,18 | 716 |
| AI926745 | 0,03 | -0,33 | -0,22 | 0,18 | 0,16 | 717 |
| R99595 | 0,05 | -0,29 | -0.19 | 0.16 | 0,20 | 718 |
| AI824579 | 0,03 | -0,31 | -0,21 | 0,13 | 0,18 | 719 |
| AA427886 | 0,03 | -0,27 | -0,17 | 0,14 | 0,16 | 720 |
| H42488 | 0,04 | -0,33 | -0,24 | 0,16 | 0,15 | 721 |

**Tabelle 3: Signifikant reduzierte Genexpressionen in Proben von Patienten mit infektiösem MODS/MOV, im Vergleich zu den Genexpressionen von Patienten mit nichtinfektiösem MODS/MOV**

| **GenBank Accession-Number** | **p-Wert** | **Mittlerer normalisierter und transformierter Expressionswert** | | **Standardabweichung** | | **Sequenz-ID** |
|---|---|---|---|---|---|---|
| | | **Gruppe der Patienten mit nichtinfektiösem /MOV** | **Gruppe der Patienten mit infektiösem MOV** | **Gruppe der Patienten mit nichtinfektiösem /MOV** | **Gruppe der Patienten mit infektiösem MOV** | |
| NM_019111 | 0,00 | 1,41 | 0,21 | 0,73 | 0,56 | 722 |
| N29761 | 0,00 | -0,25 | -1,35 | 0,61 | 0,92 | 723 |
| NM_002124 | 0,00 | 1,60 | 0,54 | 0,62 | 0,52 | 724 |
| R43910 | 0,00 | 2.51 | 1,49 | 1,25 | 0,88 | 725 |
| NM_000570 | 0,00 | 3,66 | 2,67 | 0,70 | 1,31 | 726 |
| NM_002923 | 0,00 | 2.03 | 1,07 | 0,83 | 0,89 | 727 |
| X00457 | 0,00 | 1,46 | 0,50 | 0,84 | 0.60 | 728 |
| NM_022555 | 0,00 | 1,86 | 0,91 | 0,58 | 0,54 | 729 |
| NM_002125 | 0,00 | 1,38 | 0.46 | 0,55 | 0,45 | 730 |
| AA620760 | 0,00 | 0,30 | -0,62 | 0,47 | 0,60 | 731 |
| NM_000569 | 0.01 | 3,13 | 2,26 | 0,86 | 1,21 | 732 |
| NM_021983 | 0,00 | 1,38 | 0,52 | 0,48 | 0,41 | 733 |
| R43203 | 0,00 | 2,03 | 1,18 | 1,16 | 0.74 | 734 |
| NM_033554 | 0,00 | 1,42 | 0,60 | 0.60 | 0,52 | 735 |
| AA626239 | 0,00 | 0,15 | -0,65 | 0.74 | 0,73 | 736 |
| NM_007328 | 0,00 | -0.31 | -1,10 | 0,49 | 0,64 | 737 |
| M90746 | 0,02 | 3,67 | 2,89 | 0,74 | 1,37 | 738 |
| T91086 | 0,00 | -0,81 | -1.59 | 0,63 | 0,67 | 739 |
| AA151104 | 0,00 | 0.11 | -0,64 | 0,46 | 0,46 | 740 |
| H45298 | 0,01 | 1,84 | 1,09 | 0.90 | 0,97 | 741 |
| NM_031311 | 0,00 | 0,78 | 0,03 | 0,52 | 0.39 | 742 |
| AI590144 | 0.00 | 1,70 | 0,96 | 0.97 | 0.70 | 743 |
| NM_001824.2 | 0,00 | 1,63 | 0,88 | 0,58 | 0,67 | 744 |
| NM_018643 | 0,00 | 1,70 | 0,96 | 0,54 | 0,66 | 745 |
| AA400790 | 0,00 | 0,93 | 0.20 | 0.59 | 0,52 | 746 |
| NM_001251 | 0,00 | 1,18 | 0,47 | 0,42 | 0,48 | 747 |
| NM_000887.2 | 0,00 | 0,24 | -0,48 | 0,92 | 0.61 | 748 |
| AI696291 | 0,00 | 0,74 | 0,04 | 0,64 | 0,39 | 749 |
| NM_031477 | 0,02 | 1,89 | 1,19 | 1,02 | 1,06 | 750 |
| AA910846 | 0,00 | 1,29 | 0,60 | 0.87 | 0,45 | 751 |
| NM_005538 | 0,00 | 1,59 | 0,91 | 0,90 | 0,72 | 752 |
| AA398331 | 0,00 | 0,13 | -0.53 | 0,51 | 0,46 | 753 |
| NM_025139 | 0,04 | -1,79 | -2,41 | 0,75 | 1,17 | 754 |
| AA398611 | 0,00 | 1,16 | 0,54 | 0,77 | 0,43 | 755 |
| NM_006682 | 0.00 | -0,05 | -0,67 | 0,46 | 0,39 | 756 |
| X52473 | 0,00 | 1,71 | 1,09 | 0,59 | 0,74 | 757 |
| AI859777 | 0,01 | -1,02 | -1.63 | 0,87 | 0,77 | 758 |
| H18649 | 0,00 | -0,41 | -1,01 | 0,30 | 0,58 | 759 |
| AI700444 | 0.00 | 1,57 | 0,97 | 0,70 | 0,63 | 760 |
| XM_001472 | 0,00 | -0.36 | -0,95 | 0,61 | 0,59 | 761 |
| XM_049959 | 0,01 | 210 | 1,53 | 0,73 | 0,78 | 762 |
| AA863064 | 0,03 | 0,96 | 0.39 | 1,20 | 0,63 | 763 |
| H88328 | 0,01 | -1,27 | -1,84 | 0,73 | 0,69 | 764 |
| R40861 | 0,00 | 0.82 | 0,25 | 0,82 | 0,51 | 765 |
| AI733498 | 0,00 | -0,37 | -0.93 | 0,36 | 0,58 | 766 |
| NM_002621 | 0,01 | 1,16 | 0.61 | 0,63 | 0,70 | 767 |
| AI732971 | 0,00 | 0,46 | -0,09 | 0,55 | 0,35 | 768 |
| AA813145 | 0,00 | 0.48 | -0,07 | 0,40 | 0,41 | 769 |
| NM_004221.2 | 0,02 | 2,05 | 1,51 | 0,75 | 0,79 | 770 |
| AA740907 | 0,00 | 0,05 | -0,49 | 0,44 | 0,32 | 771 |
| NM_032022 | 0,00 | 0,90 | 0,36 | 0,42 | 0,50 | 772 |
| XM_003789 | 0,00 | -0,25 | -0,78 | 0,37 | 0,47 | 773 |
| AI357099 | 0,02 | -1,06 | -1,59 | 0,85 | 0,78 | 774 |
| NM_003937 | 0.00 | -0,43 | -0,95 | 0,44 | 0,44 | 775 |
| NM_002122 | 0,00 | 0,84 | 0,33 | 0,71 | 0,51 | 776 |
| AI625626 | 0,01 | 0.90 | 0,40 | 0,87 | 0,52 | 777 |
| H23819 | 0.00 | 0.97 | 0,46 | 0,62 | 0,47 | 778 |
| AI797009 | 0,01 | 0,64 | 0,14 | 0,85 | 0,57 | 779 |
| XM_031354 | 0,01 | 0.99 | 0,49 | 0,63 | 0,72 | 780 |
| XM_051958 | 0,01 | 1,20 | 0,70 | 0,53 | 0,72 | 781 |
| AI499173 | 0,01 | 0.11 | -0,38 | 0,75 | 0,50 | 782 |
| NM_000591 | 0,00 | 0.92 | 0,43 | 0,50 | 0,55 | 783 |
| NM_057158 | 0,00 | -0,03 | -0,52 | 0,49 | 0,34 | 784 |
| R71775 | 0,00 | 0,42 | -0.07 | 0,61 | 0,55 | 785 |
| AI924028 | 0,00 | -0,35 | -0,84 | 0,36 | 0,58 | 786 |
| R39504 | 0,00 | -0,50 | -0,98 | 0,34 | 0,40 | 787 |
| N66205 | 0,01 | 1,49 | 1,02 | 0,72 | 0,62 | 788 |
| AI738831 | 0,00 | 0,09 | -0,38 | 0,29 | 0,56 | 789 |
| H18435 | 0,00 | 0,34 | -0,14 | 0,43 | 0,32 | 790 |
| R39782 | 0,00 | -0,35 | -0,82 | 0,44 | 0,35 | 791 |
| R38717 | 0,00 | -0,16 | -0,63 | 0,43 | 0,48 | 792 |
| H96798 | 0,00 | 0,19 | -0,27 | 0,47 | 0,53 | 793 |
| N72174 | 0,02 | 0,92 | 0,46 | 0,53 | 0,73 | 794 |
| AI739381 | 0,00 | -0,11 | -0,57 | 0,24 | 0,50 | 795 |
| AI654546 | 0,00 | 0,05 | -0,41 | 0,31 | 0,46 | 796 |
| AI097494 | 0.00 | -0.72 | -1,19 | 0,41 | 0,57 | 797 |
| NM_000612.2 | 0,00 | 0,93 | 0,47 | 0,50 | 0,36 | 798 |
| AI651536 | 0,00 | 0,62 | 0,16 | 0,50 | 0,29 | 799 |
| AI804425 | 0,00 | 0,90 | 0,44 | 0,73 | 0,38 | 800 |
| n67686 | 0,00 | 0,43 | -0,03 | 0,62 | 0,38 | 801 |
| NM_000062 | 0,01 | -0,18 | -0,63 | 0,75 | 0,45 | 802 |
| R54442 | 0,00 | 0,69 | 0,24 | 0,53 | 0,39 | 803 |
| AI475085 | 0,00 | 0.25 | -0,20 | 0,67 | 0,29 | 804 |
| AI700612 | 0,01 | -0,85 | -1,30 | 0,60 | 0,61 | 805 |
| AA447615 | 0,00 | 0,18 | -0,27 | 0,60 | 0,30 | 806 |
| AI223092 | 0,00 | -0,38 | -0,82 | 0,30 | 0,56 | 807 |
| AI262894 | 0,00 | 0,47 | 0,03 | 0,61 | 0,39 | 808 |
| R52949 | 0,01 | -1,08 | -1,52 | 0,44 | 0,72 | 809 |
| AA629034 | 0,00 | -0,13 | -0,57 | 0,37 | 0,33 | 810 |
| R12559 | 0,00 | 0,72 | 0,29 | 0,45 | 0.37 | 811 |
| AA910310 | 0,00 | 0,03 | -0,40 | 0,35 | 0,27 | 812 |
| NM_006850 | 0,01 | 0,19 | -0,24 | 0,59 | 0,51 | 813 |
| AI689080 | 0,05 | 1,34 | 0,91 | 0,74 | 0,78 | 814 |
| R23755 | 0,00 | 0.16 | -0.26 | 0,40 | 0,37 | 815 |
| N95041 | 0,00 | 0,17 | -0,25 | 0,30 | 0,40 | 816 |
| AA443712 | 0,01 | 0,76 | 0,34 | 0,72 | 0,41 | 817 |
| NM_033302 | 0,03 | 1,53 | 1,11 | 0,54 | 0,72 | 818 |
| AI700810 | 0,00 | 0,59 | 0,18 | 0,72 | 0,25 | 819 |
| XM_004011 | 0,00 | 0,52 | 0,11 | 0,44 | 0,35 | 820 |
| H11433 | 0,00 | 0,38 | -0,03 | 0,55 | 0,35 | 821 |
| NM_006890 | 0.03 | 1,14 | 0,73 | 0,77 | 0,52 | 822 |
| NM_138556 | 0,00 | 0,16 | -0,25 | 0,23 | 0,36 | 823 |
| XM_003937 | 0,00 | 0,13 | -0,28 | 0,34 | 0,33 | 824 |
| NM_000908.1 | 0,00 | -0, 05 | -0,46 | 0,22 | 0,30 | 825 |
| NM_017567 | 0,01 | -0,52 | -0,92 | 0,57 | 0,47 | 826 |
| R89802 | 0,00 | -0,21 | -0,61 | 0,27 | 0,33 | 827 |
| NM_000715 | 0,01 | 0,77 | 0,37 | 0,56 | 0,46 | 828 |
| AI924733 | 0,00 | -0,60 | -1,00 | 0,37 | 0, 50 | 829 |
| AI859370 | 0,00 | 0,17 | -0,23 | 0,16 | 0,24 | 830 |
| AI023558 | 0,00 | -0.41 | -0,80 | 0,23 | 0,37 | 831 |
| AA021303 | 0,00 | 0,19 | -0,20 | 0,58 | 0,25 | 832 |
| R69609 | 0,01 | 1,03 | 0,64 | 0,54 | 0,51 | 833 |
| XM_057445 | 0,00 | 0,27 | -0,12 | 0,35 | 0,39 | 834 |
| AA046302 | 0.00 | -0.10 | -0,49 | 0,36 | 0,33 | 835 |
| AI383451 | 0.01 | 0,26 | -0.13 | 0,53 | 0,40 | 836 |
| AA464191 | 0,00 | -0,46 | -0,84 | 0,32 | 0,41 | 837 |
| AA425808 | 0,00 | -0,22 | -0,61 | 0,25 | 0,51 | 838 |
| XM_038024 | 0,00 | 0,18 | -0,21 | 0,28 | 0,47 | 839 |
| AI016127 | 0,01 | 1,07 | 0,69 | 0,56 | 0,42 | 840 |
| AA400144 | 0,03 | -0,41 | -0,79 | 0,60 | 0,62 | 841 |
| R43074 | 0,00 | -0,99 | -1,36 | 0,28 | 0.51 | 842 |
| AI628936 | 0,01 | -0,65 | -1,03 | 0,41 | 0,51 | 843 |
| AA461499 | 0,00 | -0,16 | -0,54 | 0,39 | 0,38 | 844 |
| AI668673 | 0.00 | 0,34 | -0,03 | 0,35 | 0,50 | 845 |
| AI539443 | 0,00 | 0,24 | -0,13 | 0,39 | 0,43 | 846 |
| AA404231 | 0,04 | -0,14 | -0,52 | 0,52 | 0,69 | 847 |
| AI692869 | 0,01 | 0,72 | 0,34 | 0,30 | 0,53 | 848 |
| AI822099 | 0,00 | 0,00 | -0,37 | 0,51 | 0,34 | 849 |
| R20616 | 0,00 | 0,12 | -0,25 | 0,30 | 0,32 | 850 |
| AA453406 | 0,01 | -0,66 | -1,03 | 0,42 | 0,49 | 851 |
| AA282404 | 0,02 | 0,07 | -0,29 | 0,46 | 0,58 | 852 |
| AI023336 | 0,00 | 0,24 | -0,13 | 0,28 | 0,27 | 853 |
| NM_001964 | 0,02 | -0,63 | -0,99 | 0,56 | 0,53 | 854 |
| N35603 | 0,04 | -0,51 | -0,87 | 0,51 | 0,67 | 855 |
| AI632210 | 0,00 | 0,35 | -0,01 | 0,60 | 0,26 | 856 |
| AA156454 | 0,00 | 0,37 | 0,01 | 0,34 | 0,35 | 857 |
| AA620836 | 0,02 | 0,24 | -0,12 | 0,51 | 0,55 | 858 |
| NM_020530 | 0,00 | 0,37 | 0,01 | 0,44 | 0,30 | 859 |
| AA928277 | 0,00 | -0,10 | -0,46 | 0,34 | 0,36 | 860 |
| NM_001559 | 0,04 | 0,37 | 0,01 | 0,73 | 0,50 | 861 |
| AA401691 | 0,00 | -0,08 | -0,44 | 0,39 | 0,38 | 862 |
| NM_015991 | 0,00 | 0,01 | -0,34 | 0,46 | 0,33 | 863 |
| N80764 | 0,00 | -0,08 | -0,43 | 0,33 | 0,43 | 864 |
| L34657 | 0,00 | 0,12 | -0,23 | 0,31 | 0,34 | 865 |
| H98244 | 0,00 | 0,24 | -0,11 | 0,39 | 0,35 | 866 |
| AA894523 | 0,00 | -0,24 | -0,59 | 0,23 | 0,29 | 867 |
| NM_013261.1 | 0,00 | 0,08 | -0,26 | 0,32 | 0,37 | 868 |
| H02254 | 0,01 | -0,39 | -0,73 | 0,40 | 0,45 | 869 |
| NM_003781.2 | 0,01 | -0,64 | -0,98 | 0,50 | 0,36 | 870 |
| NM_001243 | 0,05 | 0,78 | 0,44 | 0,51 | 0,66 | 871 |
| AA442897 | 0,01 | -0,44 | -0,78 | 0,32 | 0,46 | 872 |
| T85314 | 0,01 | -0,29 | -0,63 | 0,46 | 0,43 | 873 |
| AI658519 | 0,05 | 0,50 | 0,16 | 0,70 | 0,50 | 874 |
| AI207975 | 0,00 | -0,28 | -0,62 | 0,37 | 0,30 | 875 |
| AI536602 | 0,00 | 0,28 | -0,06 | 0,47 | 0,33 | 876 |
| NM_001541.1 | 0,00 | 0,50 | 0,16 | 0,38 | 0,27 | 877 |
| AA992540 | 0,00 | 0,14 | -0,19 | 0,31 | 0,32 | 878 |
| Z22971 | 0,01 | 0,62 | 0,29 | 0,51 | 0,39 | 879 |
| AI560847 | 0,00 | 0,36 | 0,03 | 0,23 | 0,28 | 880 |
| XM_008346 | 0,04 | 0,40 | 0,07 | 0,59 | 0,54 | 881 |
| AA015795 | 0,02 | -0,36 | -0,69 | 0,57 | 0,42 | 882 |
| R00742 | 0,00 | 0,37 | 0,04 | 0,34 | 0,33 | 883 |
| H16774 | 0,00 | 0,02 | -0,31 | 0,33 | 0,24 | 884 |
| R51373 | 0,00 | 0,15 | -0,18 | 0,31 | 0,24 | 885 |
| AI479659 | 0,00 | 0,18 | -0,14 | 0,34 | 0,29 | 886 |
| W58195 | 0,00 | -0,06 | -0,39 | 0,27 | 0,39 | 887 |
| NM_00437.1 | 0,05 | 1,06 | 0,73 | 0,47 | 0,65 | 888 |
| AA479357 | 0,00 | 0,18 | -0,15 | 0,30 | 0,21 | 889 |
| AI423518 | 0,00 | -0,25 | -0,57 | 0,29 | 0,40 | 890 |
| NM_002750 | 0,01 | -0,52 | -0,85 | 0,36 | 0,44 | 891 |
| R26444 | 0,00 | 0,00 | -0,32 | 0,27 | 0,36 | 892 |
| AA136071 | 0,00 | 0,04 | -0,29 | 0,25 | 0,34 | 893 |
| AI554459 | 0,00 | 0,02 | -0,34 | 0,39 | 0,35 | 894 |
| N51537 | 0,02 | 0,89 | 0,57 | 0,45 | 0,49 | 895 |
| NM_006068 | 0,00 | 0,62 | 0,30 | 0,35 | 0,39 | 896 |
| NM_016184 | 0,03 | 0,61 | 0,29 | 0,49 | 0,52 | 897 |
| NM_000586 | 0,03 | 0,03 | -0,29 | 0,40 | 0,54 | 898 |
| NM_003102.1 | 0,01 | -0,39 | -0,71 | 0,49 | 0,43 | 899 |
| AI264774 | 0,00 | -0,11 | -0,43 | 0,20 | 0,44 | 900 |
| N90536 | 0,01 | -0,45 | -0,77 | 0,30 | 0,45 | 901 |
| AA404342 | 0,00 | -0,29 | -0,61 | 0,36 | 0,36 | 902 |
| AI373525 | 0,00 | -0,16 | -0,48 | 0,30 | 0,25 | 903 |
| AI579907 | 0,00 | 0,07 | -0,25 | 0,38 | 0,25 | 904 |
| AA279410 | 0,00 | 0,11 | -0,21 | 0,33 | 0,26 | 905 |
| XM_038308 | 0,04 | 0,35 | 0,03 | 0,51 | 0,54 | 906 |
| NM_000879 | 0,02 | -0,01 | -0,33 | 0,37 | 0,52 | 907 |
| NM_001078) | 0,00 | 0,38 | 0,07 | 0,38 | 0,32 | 908 |
| AA781411 | 0,00 | -0,24 | -0,55 | 0,23 | 0,37 | 909 |
| R07171 | 0,00 | -0,16 | -0,48 | 0,34 | 0,37 | 910 |
| AA136273 | 0,00 | -0,10 | -0,41 | 0,26 | 0,32 | 911 |
| AI565469 | 0,01 | -0,06 | -0,37 | 0,32 | 0,41 | 912 |
| AI799767 | 0,00 | -0,12 | -0,44 | 0,35 | 0,36 | 913 |
| AI889554 | 0,00 | -0,08 | -0,39 | 0,34 | 0,36 | 914 |
| AA410301 | 0,01 | 0,77 | 0,46 | 0,35 | 0,42 | 915 |
| AA995114 | 0,04 | 1,09 | 0,79 | 0,67 | 0,40 | 916 |
| AI694444 | 0,00 | -0,40 | -0,71 | 0,26 | 0,35 | 917 |
| T98940 | 0,00 | 0,05 | -0,26 | 0,45 | 0,27 | 918 |
| R16722 | 0,00 | 0,07 | -0,23 | 0,42 | 0,23 | 919 |
| H05436 | 0,00 | 0,40 | 0,10 | 0,34 | 0,33 | 920 |
| R42778 | 0,01 | 0,39 | 0,09 | 0,45 | 0,33 | 921 |
| AI378275 | 0,00 | -0,02 | -0,33 | 0,29 | 0,40 | 922 |
| XM_083833 | 0,03 | 0,50 | 0,20 | 0,57 | 0,39 | 923 |
| R94894 | 0,03 | 1,00 | 0,70 | 0,35 | 0,55 | 924 |
| H15677 | 0,01 | -0,24 | -0,54 | 0,34 | 0,45 | 925 |
| AI625523 | 0,04 | 0,75 | 0,45 | 0,47 | 0,51 | 926 |
| AI627286 | 0,00 | 0,03 | -0,27 | 0,35 | 0,26 | 927 |
| NM_003807 | 0,01 | 0,08 | -0,22 | 0,35 | 0,42 | 928 |
| NM_002757 | 0,02 | 0,00 | -0,30 | 0,50 | 0,41 | 929 |
| XM_008411 | 0,02 | -0,47 | -0,77 | 0,31 | 0,51 | 930 |
| AI379294 | 0,01 | -0,06 | -0,35 | 0,45 | 0,32 | 931 |
| AI824470 | 0,00 | -0,20 | -0,49 | 0,19 | 0,42 | 932 |
| N94525 | 0,00 | 0,15 | -0,14 | 0,26 | 0,28 | 933 |
| R38432 | 0,01 | -0,02 | -0,32 | 0,27 | 0,41 | 934 |
| NM_017436.2 | 0,02 | -0,44 | -0,74 | 0,42 | 0,44 | 935 |
| AA398968 | 0,00 | -0,03 | -0,32 | 0,37 | 0,35 | 936 |
| U15085 | 0,03 | -0,89 | -1,18 | 0,47 | 0,47 | 937 |
| AI734941 | 0,01 | -0,14 | -0,43 | 0,31 | 0,41 | 938 |
| AI819159 | 0,00 | 0,44 | 0,15 | 0,39 | 0,28 | 939 |
| AA426024 | 0,02 | -0,11 | -0,40 | 0,46 | 0,42 | 940 |
| AA435854 | 0,00 | -0,33 | -0,62 | 0,21 | 0,28 | 941 |
| NM_003264 | 0,00 | 0,28 | -0,01 | 0,30 | 0,38 | 942 |
| NM 001622.1 | 0,04 | 0,01 | -0,28 | 0,41 | 0,53 | 943 |
| AI828714 | 0,04 | -0,25 | -0,55 | 0,33 | 0,54 | 944 |
| NM 006610 | 0,00 | -0,04 | -0,33 | 0,23 | 0,30 | 945 |
| AI143013 | 0,00 | -0,04 | -0,33 | 0,38 | 0,31 | 946 |
| AA428992 | 0,01 | 0,50 | 0,21 | 0,48 | 0,24 | 947 |
| R40560 | 0,02 | 0,17 | -0,12 | 0,33 | 0,44 | 948 |
| AI203091 | 0,02 | -0,44 | -0,73 | 0,28 | 0,50 | 949 |
| T92041 | 0,00 | 0,07 | -0,22 | 0,28 | 0,22 | 950 |
| AA453794 | 0,00 | 0,20 | -0,09 | 0,22 | 0,29 | 951 |
| R05804 | 0,00 | 0,18 | -0,11 | 0,22 | 0,34 | 952 |
| AA453489 | 0,01 | -0,56 | -0,85 | 0,33 | 0,37 | 953 |
| NM 006664 | 0,00 | 0,67 | 0,39 | 0,30 | 0,35 | 954 |
| AA281330 | 0,03 | 0,76 | 0,48 | 0,57 | 0,38 | 955 |
| AA452139 | 0,00 | 0,08 | -0,20 | 0,31 | 0,24 | 956 |
| R43204 | 0,00 | 0,19 | -0,09 | 0,38 | 0,21 | 957 |
| NM_012340 | 0,01 | 0,05 | -0,24 | 0,36 | 0,40 | 958 |
| NM_004778 | 0,02 | 0,00 | -0,28 | 0,43 | 0,40 | 959 |
| AA490815 | 0,01 | 0,04 | -0,24 | 0,26 | 0,44 | 960 |
| NM_022740 | 0,00 | 0,47 | 0,19 | 0,30 | 0,31 | 961 |
| AI167874 | 0,01 | 0,33 | 0,05 | 0,41 | 0,33 | 962 |
| AA149968 | 0,00 | -0,09 | -0,37 | 0,28 | 0,27 | 963 |
| XM_058179 | 0,03 | -0,04 | -0,32 | 0,58 | 0,35 | 964 |
| R07502 | 0,00 | -0,42 | -0,70 | 0,33 | 0,31 | 965 |
| NM_000752 | 0,01 | -0,27 | -0,56 | 0,48 | 0,29 | 966 |
| XM_003529 | 0,01 | 0,22 | -0,06 | 0,42 | 0,38 | 967 |
| N64541 | 0,01 | 0,13 | -0,15 | 0,44 | 0,37 | 968 |
| NM_001054 | 0,01 | 0,18 | -0,10 | 0,32 | 0,40 | 969 |
| AI499407 | 0,00 | 0,00 | -0,28 | 0,30 | 0,27 | 970 |
| NM_020056 | 0,00 | -0,05 | -0,33 | 0,32 | 0,28 | 971 |
| AA004952 | 0,01 | -0,20 | -0,48 | 0,41 | 0,31 | 972 |
| AI624610 | 0,01 | 0,09 | -0,19 | 0,34 | 0,38 | 973 |
| AA421924 | 0,04 | 0,92 | 0,64 | 0,49 | 0,44 | 974 |
| AI732550 | 0,04 | 0,03 | -0,25 | 0,51 | 0,43 | 975 |
| AI374599 | 0,02 | -0,15 | -0,43 | 0,24 | 0,47 | 976 |
| AI582909 | 0,00 | 0,34 | 0,06 | 0,21 | 0,21 | 977 |
| AI554111 | 0,00 | 0,21 | -0,07 | 0,39 | 0,21 | 978 |
| NM_001734 | 0,00 | -0,21 | -0,49 | 0,21 | 0,37 | 979 |
| AA810014 | 0,03 | 0,23 | -0,05 | 0,56 | 0,33 | 980 |
| AI373295 | 0,00 | 0,32 | 0,05 | 0,31 | 0,23 | 981 |
| XM_048555 | 0,01 | -0,20 | -0,48 | 0,38 | 0,34 | 982 |
| AA435627 | 0,00 | 0,15 | -0,13 | 0,31 | 0,26 | 983 |
| T95815 | 0,00 | 0,55 | 0,27 | 0,33 | 0,32 | 984 |
| AA426030 | 0,03 | -0,14 | -0,42 | 0,40 | 0,42 | 985 |
| AI720051 | 0,01 | -0,29 | -0,56 | 0,30 | 0,43 | 986 |
| AI278521 | 0,01 | -0,50 | -0,77 | 0,39 | 0,34 | 987 |
| N93236 | 0,01 | 0,38 | 0,10 | 0,38 | 0,34 | 988 |
| NM_015645 | 0,03 | -0,28 | -0,55 | 0,44 | 0,43 | 989 |
| AI671360 | 0,00 | 0,22 | -0,05 | 0,28 | 0,28 | 990 |
| T83666 | 0,00 | 0,13 | -0,14 | 0,36 | 0,21 | 991 |
| WO2063 | 0,00 | -0,02 | -0,30 | 0,31 | 0,31 | 992 |
| AI659563 | 0,00 | 0,01 | -0,26 | 0,27 | 0,21 | 993 |
| NM_139046 | 0,02 | -0,47 | -0,74 | 0,35 | 0,45 | 994 |
| AA155745 | 0,00 | 0,00 | -0,27 | 0,31 | 0,26 | 995 |
| H40035 | 0,01 | -0,32 | -0,59 | 0,28 | 0,38 | 996 |
| AA101379 | 0,00 | 0,26 | -0,02 | 0,35 | 0,31 | 997 |
| H16790 | 0,00 | 0,22 | -0,05 | 0,37 | 0,28 | 998 |
| AA011511 | 0,02 | -0,29 | -0,55 | 0,32 | 0,41 | 999 |
| AA746495 | 0,05 | 0,17 | -0,10 | 0,56 | 0,39 | 1000 |
| AA845015 | 0,00 | -0,04 | -0,30 | 0,34 | 0,26 | 1001 |
| NM_138636 | 0,05 | 0,51 | 0,24 | 0,39 | 0,52 | 1002 |
| NM_033358 | 0,01 | 0,50 | 0,24 | 0,37 | 0,37 | 1003 |
| AI650349 | 0,02 | -0,13 | -0,39 | 0,40 | 0,41 | 1004 |
| NM_001764 | 0,01 | 0,33 | 0,06 | 0,46 | 0,20 | 1005 |
| XM_006447 | 0,03 | -0,53 | -0,80 | 0,49 | 0,39 | 1006 |
| R07185 | 0,00 | 0,12 | -0,14 | 0,34 | 0,22 | 1007 |
| AA187437 | 0,00 | -0,01 | -0,27 | 0,21 | 0,26 | 1008 |
| AI621365 | 0,00 | 0,25 | -0,02 | 0,34 | 0,28 | 1009 |
| NM_020205 | 0,03 | 0,16 | -0,10 | 0,29 | 0,48 | 1010 |
| AI888390 | 0,01 | -0,89 | -1,15 | 0,31 | 0,40 | 1011 |
| AI674699 | 0,01 | -0,09 | -0,35 | 0,34 | 0,37 | 1012 |
| AI620249 | 0,02 | 0,02 | -0,24 | 0,49 | 0,27 | 1013 |
| NM_033295 | 0,02 | -0,32 | -0,58 | 0,41 | 0,39 | 1014 |
| NM_015718.1 | 0,00 | -0,08 | -0,34 | 0,23 | 0,34 | 1015 |
| N73572 | 0,05 | 0,05 | -0,21 | 0,45 | 0,42 | 1016 |
| AI420037 | 0,02 | 0,04 | -0,22 | 0,46 | 0,31 | 1017 |
| AI684431 | 0,00 | 0,28 | 0,03 | 0,32 | 0,27 | 1018 |
| AA017263 | 0,00 | 0,11 | -0,14 | 0,38 | 0,25 | 1019 |
| R45118 | 0,01 | 0,16 | -0,10 | 0,32 | 0,33 | 1020 |
| AI267659 | 0,04 | 0,01 | -0,25 | 0,21 | 0,53 | 1021 |
| AA406083 | 0,03 | 0,00 | -0,26 | 0,38 | 0,41 | 1022 |
| W48664 | 0,00 | 0,21 | -0,05 | 0,31 | 0,22 | 1023 |
| AA514450 | 0,00 | -0,38 | -0,63 | 0,26 | 0,33 | 1024 |
| AI150305 | 0,00 | 0,30 | 0,04 | 0,23 | 0,32 | 1025 |
| AA481504 | 0,03 | -0,74 | -0,99 | 0,37 | 0,42 | 1026 |
| R44840 | 0,02 | 0,22 | -0,04 | 0,45 | 0,32 | 1027 |
| AI160757 | 0,00 | 0,21 | -0,05 | 0,29 | 0,29 | 1028 |
| AA040870 | 0,00 | 0,24 | -0,02 | 0,30 | 0,30 | 1029 |
| AI342905 | 0,02 | 0,49 | 0,24 | 0,43 | 0,35 | 1030 |
| N68463 | 0,05 | 0,09 | -0,16 | 0,46 | 0,43 | 1031 |
| AA398760 | 0,00 | 0,05 | -0,20 | 0,24 | 0,23 | 1032 |
| AI798514 | 0,00 | 0,26 | 0,00 | 0,30 | 0,25 | 1033 |
| AI081725 | 0,00 | 0,18 | -0,07 | 0,31 | 0,28 | 1034 |
| AI799385 | 0,03 | 0,44 | 0,19 | 0,45 | 0,37 | 1035 |
| AA897543 | 0,04 | -0,24 | -0,49 | 0,28 | 0,49 | 1036 |
| N79807 | 0,01 | 0,18 | -0,07 | 0,33 | 0,33 | 1037 |
| AI676097 | 0,05 | 0,21 | -0,04 | 0,57 | 0,32 | 1038 |
| R46372 | 0,01 | 0,02 | -0,23 | 0,28 | 0,37 | 1039 |
| AA448817 | 0,00 | 0,26 | 0,01 | 0,28 | 0,27 | 1040 |
| AI810161 | 0,01 | 0,09 | -0,16 | 0,31 | 0,38 | 1041 |
| H80437 | 0,00 | 0,18 | -0,07 | 0,23 | 0,29 | 1042 |
| AA443664 | 0,00 | -0,02 | -0,27 | 0,29 | 0,27 | 1043 |
| NM_002957.3 | 0,01 | -0,12 | -0,37 | 0,24 | 0,37 | 1044 |
| N69363 | 0,03 | -0,35 | -0,59 | 0,37 | 0,40 | 1045 |
| NM_000552.2 | 0,01 | -0,11 | -0,36 | 0,25 | 0,34 | 1046 |
| AA455080 | 0,01 | 0,08 | -0,16 | 0,34 | 0,28 | 1047 |
| W32272 | 0,00 | -0,25 | -0,50 | 0,26 | 0,30 | 1048 |
| H38087 | 0,04 | 0,76 | 0,51 | 0,34 | 0,47 | 1049 |
| AA504336 | 0,01 | 0,26 | 0,02 | 0,32 | 0,33 | 1050 |
| H04977 | 0,00 | 0,45 | 0,21 | 0,28 | 0,28 | 1051 |
| NM_002670 | 0,05 | 0,19 | -0,06 | 0,32 | 0,50 | 1052 |
| R09417 | 0,02 | -0,07 | -0,32 | 0,31 | 0,41 | 1053 |
| AA040057 | 0,02 | -0,05 | -0,29 | 0,35 | 0,37 | 1054 |
| AI263210 | 0,01 | -0,10 | -0,34 | 0,27 | 0,33 | 1055 |
| AI264626 | 0,01 | -0,12 | -0,37 | 0,33 | 0,29 | 1056 |
| AI478847 | 0,02 | 0,11 | -0,13 | 0,34 | 0,40 | 1057 |
| AI744042 | 0,03 | -0,37 | -0,61 | 0,51 | 0,27 | 1058 |
| AA682790 | 0,02 | 0,01 | -0,23 | 0,32 | 0,40 | 1059 |
| AA629051 | 0,01 | 0,28 | 0,04 | 0,32 | 0,29 | 1060 |
| AI560242 | 0.02 | -0,23 | -0,47 | 0,36 | 0,34 | 1061 |
| AA035428 | 0,01 | -0,14 | -0,38 | 0,26 | 0,32 | 1062 |
| NM_014326 | 0,02 | 0,11 | -0,13 | 0,52 | 0,15 | 1063 |
| AI632740 | 0,01 | -0,16 | -0,40 | 0,31 | 0,30 | 1064 |
| AI130878 | 0,01 | 0,27 | 0,03 | 0,32 | 0,31 | 1065 |
| AI933013 | 0,01 | 0,31 | 0.07 | 0,35 | 0,26 | 1066 |
| AI086719 | 0,01 | 0,00 | -0,24 | 0,37 | 0,24 | 1067 |
| R16568 | 0,03 | 0,10 | -0,14 | 0,24 | 0,46 | 1068 |
| AA009562 | 0,01 | -0,20 | -0,44 | 0,28 | 0,33 | 1069 |
| AI015069 | 0,01 | 0,04 | -0,20 | 0,32 | 0,34 | 1070 |
| AA291486 | 0,02 | -0,26 | -0,49 | 0,31 | 0,36 | 1071 |
| H65288 | 0,03 | -0,13 | -0,37 | 0,26 | 0,46 | 1072 |
| W86767 | 0,02 | 0,07 | -0,17 | 0,20 | 0,42 | 1073 |
| H65331 | 0,01 | 0,55 | 0,31 | 0,33 | 0,33 | 1074 |
| AA478985 | 0,04 | -0,12 | -0,36 | 0,20 | 0,51 | 1075 |
| H11274 | 0,02 | -0,02 | -0,26 | 0,28 | 0,40 | 1076 |
| AA044225 | 0,00 | -0,09 | -0,33 | 0,31 | 0,22 | 1077 |
| AI801415 | 0,00 | -0,08 | -0,32 | 0,32 | 0,23 | 1078 |
| AA846527 | 0,00 | -0,14 | -0,37 | 0.24 | 0,25 | 1079 |
| R56890 | 0,01 | -0,04 | -0,28 | 0,25 | 0,34 | 1080 |
| AI921525 | 0,03 | -0,06 | -0,29 | 0,36 | 0,40 | 1081 |
| AA405485 | 0,02 | 0,11 | -0,13 | 0,40 | 0,33 | 1082 |
| AA845635 | 0,00 | -0,03 | -0,26 | 0,31 | 0,26 | 1083 |
| AI150418 | 0,01 | 0,07 | -0,17 | 0,23 | 0,33 | 1084 |
| XM_049849 | 0,02 | 0, 55 | 0,32 | 0,32 | 0,37 | 1085 |
| AA406573 | 0,00 | 0,20 | -0,03 | 0,33 | 0,23 | 1086 |
| AA043930 | 0,01 | -0,26 | -0,49 | 0,27 | 0,35 | 1087 |
| AI125496 | 0,01 | -0,30 | -0,53 | 0,29 | 0,33 | 1088 |
| AI654739 | 0,02 | -0,06 | -0,29 | 0,31 | 0,35 | 1089 |
| AA398320 | 0,01 | -0,32 | -0,56 | 0,37 | 0,30 | 1090 |
| NM_002155 | 0,04 | 0,48 | 0,25 | 0,36 | 0,43 | 1091 |
| AA505872 | 0,01 | 0,71 | 0,48 | 0,31 | 0,34 | 1092 |
| NM_016610 | 0,02 | 0.24 | 0,00 | 0,20 | 0,43 | 1093 |
| AA703200 | 0,00 | -0,13 | -0,36 | 0,26 | 0,29 | 1094 |
| R44493 | 0,00 | 0,04 | -0,19 | 0,24 | 0, 23 | 1095 |
| XM_046575 | 0,04 | -0,14 | -0,38 | 0,40 | 0,40 | 1096 |
| AI275613 | 0,03 | 0,24 | 0,00 | 0,44 | 0,30 | 1097 |
| AI08602 | 0,04 | 0,19 | -0,05 | 0,38 | 0,40 | 1098 |
| R44328 | 0,01 | 0,24 | 0,01 | 0,30 | 0,30 | 1099 |
| R00206 | 0,00 | 0,07 | -0,16 | 0,23 | 0,31 | 1100 |
| NM_002456 | 0,01 | 0,02 | -0,21 | 0,37 | 0,25 | 1101 |
| AI699371 | 0,03 | -0,18 | -0,41 | 0.46 | 0,28 | 1102 |
| AA935135 | 0,03 | 0,21 | -0,02 | 0,41 | 0,33 | 1103 |
| AA702529 | 0,02 | 0,06 | -0,17 | 0,40 | 0,27 | 1104 |
| AI568023 | 0,02 | -0,19 | -0,42 | 0,36 | 0,30 | 1105 |
| NM_002768 | 0,01 | -0,65 | -0,88 | 0,27 | 0,31 | 1106 |
| AA687208 | 0,02 | -0,32 | -0,55 | 0,24 | 0,39 | 1107 |
| AI221524 | 0,04 | 0,47 | 0,25 | 0,49 | 0,31 | 1108 |
| AA813007 | 0,01 | 0,09 | -0,13 | 0,24 | 0,33 | 1109 |
| AA421326 | 0,02 | -0,33 | -0,55 | 0,28 | 0,38 | 1110 |
| AA922397 | 0,01 | 0,06 | -0,17 | 0,19 | 0,33 | 1111 |
| R51857 | 0,03 | 0,90 | 0,67 | 0,40 | 0,30 | 1112 |
| NM_006564 | 0,00 | -0,22 | -0,44 | 0,33 | 0,21 | 1113 |
| AA807376 | 0,01 | 0,39 | 0,17 | 0,31 | 0,25 | 1114 |
| AA812763 | 0,04 | -0,45 | -0,68 | 0,36 | 0,37 | 1115 |
| AA528169 | 0,02 | 0,34 | 0,12 | 0,37 | 0,32 | 1116 |
| AI804325 | 0,01 | -0,14 | -0,36 | 0,32 | 0,24 | 1117 |
| T70330 | 0,04 | -0,10 | -0,33 | 0,30 | 0,41 | 1118 |
| NM_001766 | 0,03 | 0,30 | 0,08 | 0,39 | 0,35 | 1119 |
| AI696956 | 0,01 | -0,12 | -0,34 | 0,40 | 0,23 | 1120 |
| AI459174 | 0,01 | -0,05 | -0,27 | 0,30 | 0,25 | 1121 |
| R35639 | 0,01 | -0,03 | -0,25 | 0,17 | 0,37 | 1122 |
| W69774 | 0,01 | -0.02 | -0,24 | 0,21 | 0,30 | 1123 |
| AA054265 | 0,05 | 0,37 | 0,15 | 0,40 | 0,38 | 1124 |
| AI382995 | 0,01 | 0,19 | -0,03 | 0,29 | 0,25 | 1125 |
| AI218303 | 0,01 | 0.00 | -0,22 | 0,31 | 0,26 | 1126 |
| AI624954 | 0,01 | -0,18 | -0,40 | 0,28 | 0,31 | 1127 |
| AA759254 | 0,05 | -0,08 | -0,30 | 0,49 | 0,29 | 1128 |
| AI682979 | 0,02 | 0,03 | -0,19 | 0,22 | 0,37 | 1129 |
| XM_001754 | 0,01 | 0,18 | -0,03 | 0,31 | 0,28 | 1130 |
| AI187401 | 0,00 | -0,01 | -0,23 | 0,21 | 0,22 | 1131 |
| AA452113 | 0,01 | 0,24 | 0,02 | 0,25 | 0,30 | 1132 |
| AI656210 | 0,04 | -0,48 | -0,70 | 0,30 | 0,40 | 1133 |
| N29999 | 0,01 | 0,21 | 0,00 | 0,22 | 0,34 | 1134 |
| N68557 | 0,01 | 0,00 | -0,21 | 0,19 | 0,32 | 1135 |
| AI689672 | 0,02 | -0,08 | -0,29 | 0,42 | 0,19 | 1136 |
| AA730310 | 0,00 | -0,07 | -0,28 | 0,25 | 0,22 | 1137 |
| AI431324 | 0,01 | -0,20 | -0,42 | 0,39 | 0,22 | 1138 |
| NM_000066 | 0,04 | -0,11 | -0,32 | 0,31 | 0,40 | 1139 |
| XM_034219 | 0,01 | 0,01 | -0,21 | 0,30 | 0,29 | 1140 |
| R43258 | 0,04 | 0,27 | 0,05 | 0,49 | 0,23 | 1141 |
| AI431293 | 0,00 | 0,07 | -0,15 | 0,25 | 0,22 | 1142 |
| R80259 | 0,04 | -0,49 | -0,70 | 0,22 | 0,38 | 1143 |
| AI126520 | 0,00 | 0,13 | -0,08 | 0,22 | 0,21 | 1144 |
| AA937226 | 0,00 | 0,02 | -0,19 | 0,25 | 0,26 | 1145 |
| AI191762 | 0,03 | -0,22 | -0,43 | 0,30 | 0,36 | 1146 |
| AA400470 | 0,00 | -0,10 | -0,31 | 0,34 | 0,17 | 1147 |
| NM_000063 | 0,01 | -0,17 | -0,38 | 0,29 | 0,23 | 1148 |
| H73962 | 0,01 | -0,11 | -0,32 | 0,22 | 0,30 | 1149 |
| AA626313 | 0,01 | -0, 06 | -0,27 | 0,23 | 0,30 | 1150 |
| AI553630 | 0,03 | 0,13 | -0,08 | 0,36 | 0,31 | 1151 |
| NM_000257.1 | 0,01 | 0,37 | 0,16 | 0,29 | 0,25 | 1152 |
| N68456 | 0,03 | 0,33 | 0,12 | 0,27 | 0,36 | 1153 |
| XM_054837 | 0,01 | 0,24 | 0,04 | 0,24 | 0,27 | 1154 |
| AI696558 | 0,04 | -0,49 | -0,70 | 0,38 | 0,33 | 1155 |
| AI299876 | 0,05 | 0,03 | -0,18 | 0,36 | 0,37 | 1156 |
| NM_006378 | 0,03 | 0,65 | 0,44 | 0,28 | 0,36 | 1157 |
| AI376955 | 0,02 | -0,56 | -0,76 | 0,31 | 0,33 | 1158 |
| AA025573 | 0,01 | -0,24 | -0,45 | 0,33 | 0,25 | 1159 |
| T99196 | 0,02 | 0,14 | -0,07 | 0,34 | 0,29 | 1160 |
| XM_005637 | 0,05 | 0,25 | 0,05 | 0,19 | 0,45 | 1161 |
| AI597729 | 0,04 | -0,01 | -0,21 | 0,24 | 0,41 | 1162 |
| H78135 | 0,02 | 0,04 | -0,17 | 0,29 | 0,33 | 1163 |
| AI695029 | 0,01 | 0,04 | -0,16 | 0,27 | 0,25 | 1164 |
| AA004279 | 0,02 | -0,18 | -0,39 | 0,21 | 0,34 | 1165 |
| AA844020 | 0,03 | 0,33 | 0,12 | 0,30 | 0,33 | 1166 |
| AI332536 | 0,00 | -0,12 | -0,33 | 0,20 | 0,18 | 1167 |
| AI383368 | 0,03 | -0,40 | -0,61 | 0,21 | 0,38 | 1168 |
| AA423883 | 0,00 | -0,06 | -0,26 | 0,17 | 0,28 | 1169 |
| R36006 | 0,02 | -0,06 | -0,26 | 0,30 | 0,29 | 1170 |
| AI911837 | 0,02 | -0,05 | -0,26 | 0,30 | 0,31 | 1171 |
| AI696820 | 0,03 | -0,37 | -0,57 | 0,32 | 0,34 | 1172 |
| H30516 | 0,02 | -0,17 | -0,37 | 0,22 | 0,34 | 1173 |
| AI926561 | 0,01 | 0,02 | -0,18 | 0,37 | 0,20 | 1174 |
| H61449 | 0,02 | -0,25 | -0,45 | 0,24 | 0,32 | 1175 |
| AA410338 | 0,02 | -0,18 | -0,38 | 0,37 | 0,26 | 1176 |
| AA485229 | 0,00 | 0,05 | -0,15 | 0,18 | 0,18 | 1177 |
| AA044828 | 0,01 | -0,01 | -0,21 | 0,22 | 0,31 | 1178 |
| R07278 | 0,03 | 0,00 | -0,20 | 0,15 | 0,39 | 1179 |
| AI687656 | 0,02 | -0,22 | -0.42 | 0,28 | 0,28 | 1180 |
| AI912316 | 0,03 | 0,21 | 0.01 | 0,42 | 0,24 | 1181 |
| AA017301 | 0,00 | -0,07 | -0,27 | 0,18 | 0,26 | 1182 |
| AA059314 | 0,05 | 0,13 | -0,07 | 0,26 | 0,40 | 1183 |
| NM_024302.2 | 0,04 | 0,20 | 0,00 | 0,29 | 0,35 | 1184 |
| AA446463 | 0,02 | -0,15 | -0,34 | 0,29 | 0,29 | 1185 |
| NM_002747 | 0.01 | 0,19 | -0,01 | 0,24 | 0,24 | 1186 |
| AA446316 | 0,02 | 0,03 | -0,17 | 0,30 | 0,29 | 1187 |
| NM_052813) | 0,05 | -0,22 | -0,42 | 0,39 | 0,30 | 1188 |
| AA731532 | 0,00 | -0,24 | -0,43 | 0,18 | 0,24 | 1189 |
| R00307 | 0,04 | 0,16 | -0,03 | 0,45 | 0,20 | 1190 |
| AI924296 | 0,03 | -0,08 | -0,28 | 0,19 | 0,36 | 1191 |
| AI017741 | 0,01 | 0,07 | -0,12 | 0,29 | 0,21 | 1192 |
| AI619681 | 0,01 | -0,18 | -0,37 | 0,17 | 0,29 | 1193 |
| AA400967 | 0,01 | 0,25 | 0,06 | 0,30 | 0,22 | 1194 |
| NM_000680.1 | 0,01 | 0,28 | 0,09 | 0,20 | 0,28 | 1195 |
| AI732878 | 0,00 | -0,09 | -0,28 | 0,16 | 0,16 | 1196 |
| XM_006454 | 0,02 | -0,08 | -0,27 | 0,34 | 0,24 | 1197 |
| AI688916 | 0,03 | 0,02 | -0,17 | 0,32 | 0,29 | 1198 |
| T79834 | 0,01 | 0,09 | -0,10 | 0,25 | 0,27 | 1199 |
| AI015693 | 0,01 | -0,01 | -0,20 | 0,20 | 0,27 | 1200 |
| R50755 | 0,00 | -0,01 | -0,20 | 0,19 | 0,19 | 1201 |
| W44337 | 0,04 | 0,05 | -0,13 | 0,18 | 0,39 | 1202 |
| H23267 | 0,03 | -0,37 | -0,55 | 0,26 | 0,31 | 1203 |
| AA101850 | 0,02 | -0,12 | -0,30 | 0,34 | 0,21 | 1204 |
| AI628322 | 0,05 | -0.03 | -0,22 | 0,37 | 0,28 | 1205 |
| R94207 | 0,02 | 0,13 | -0,06 | 0,26 | 0,28 | 1206 |
| NM_004347 | 0,03 | 0,32 | 0,14 | 0,35 | 0,27 | 1207 |
| AA960802 | 0,05 | 0,14 | -0,04 | 0,37 | 0,29 | 1208 |
| NM_052962 | 0,02 | -0,42 | -0,60 | 0,26 | 0,25 | 1209 |
| T91946 | 0,04 | 0,14 | -0,05 | 0,29 | 0,33 | 1210 |
| AA531564 | 0,04 | -0,14 | -0,32 | 0,37 | 0,26 | 1211 |
| R96155 | 0,01 | 0,00 | -0,18 | 0,28 | 0,21 | 1212 |
| AI825491 | 0,02 | -0,07 | -0,25 | 0,19 | 0,29 | 1213 |
| N53973 | 0,02 | 0,01 | -0,17 | 0,22 | 0,31 | 1214 |
| NM_001544 | 0,01 | 0,10 | -0,08 | 0,27 | 0,22 | 1215 |
| AA702731 | 0,00 | -0,16 | -0,34 | 0,19 | 0,23 | 1216 |
| AI554655 | 0,05 | -0,04 | -0,22 | 0,23 | 0,36 | 1217 |
| H17495 | 0,04 | 0,50 | 0,32 | 0,29 | 0,31 | 1218 |
| AI209185 | 0,02 | -0,24 | -0,42 | 0,16 | 0,31 | 1219 |
| AA031813 | 0,03 | -0,15 | -0,33 | 0,29 | 0,27 | 1220 |
| NM_004166 | 0,04 | -0,37 | -0,54 | 0,35 | 0,27 | 1221 |
| AA461044 | 0,02 | 0,06 | -0,11 | 0,21 | 0,31 | 1222 |
| N45328 | 0,05 | -0,12 | -0,29 | 0,32 | 0,30 | 1223 |
| N64446 | 0,03 | -0,24 | -0,42 | 0,24 | 0,32 | 1224 |
| AI633617 | 0.01 | -0,05 | -0,22 | 0,23 | 0,24 | 1225 |
| R45159 | 0,03 | 0,22 | 0,05 | 0,32 | 0,24 | 1226 |
| R60898 | 0,00 | 0,13 | -0,04 | 0,18 | 0,17 | 1227 |
| AI621170 | 0,03 | -0,05 | -0,22 | 0,30 | 0,27 | 1228 |
| N99049 | 0,01 | 0,16 | -0,01 | 0,31 | 0,19 | 1229 |
| H18651 | 0,01 | 0,19 | 0,02 | 0,24 | 0,22 | 1230 |
| AA568582 | 0,04 | 0,02 | -0,15 | 0,30 | 0,28 | 1231 |
| AA026871 | 0,03 | -0,02 | -0,19 | 0,37 | 0,20 | 1232 |
| AI559626 | 0,01 | -0,11 | -0,28 | 0,23 | 0,21 | 1233 |
| AA443545 | 0,03 | 0,46 | 0,29 | 0,27 | 0,28 | 1234 |
| R43339 | 0,04 | 0,22 | 0,06 | 0,36 | 0,23 | 1235 |
| AA007369 | 0,04 | -0,16 | -0,33 | 0,28 | 0,30 | 1236 |
| AA960982 | 0,01 | 0,25 | 0,08 | 0,26 | 0,22 | 1237 |
| AA481399 | 0,01 | 0,01 | -0,16 | 0,30 | 0,18 | 1238 |
| AA280005 | 0,02 | -0,17 | -0,34 | 0,23 | 0,26 | 1239 |
| NM_005666 | 0,01 | 0,36 | 0,19 | 0,26 | 0,20 | 1240 |
| NM_000491 | 0,03 | 0,08 | -0,09 | 0,3 | 0,24 | 1241 |
| AA844053 | 0,03 | -0,12 | -0,28 | 0,22 | 0,26 | 1242 |
| R49384 | 0,01 | -0,05 | -0,22 | 0,24 | 0,21 | 1243 |
| AI698289 | 0,01 | -0,16 | -0,33 | 0,23 | 0,21 | 1244 |
| AI680467 | 0,04 | -0,09 | -0,26 | 0,23 | 0,31 | 1245 |
| M90391 | 0,03 | -0,11 | -0,28 | 0,27 | 0,23 | 1246 |
| AF218727 | 0,05 | 0,18 | 0,02 | 0,25 | 0,31 | 1247 |
| H22946 | 0,04 | -0,44 | -0,60 | 0,27 | 0,29 | 1248 |
| N49285 | 0,03 | -0,51 | -0,67 | 0,23 | 0,28 | 1249 |
| N74903 | 0,01 | 0,17 | 0,01 | 0,21 | 0,22 | 1250 |
| NM_001066.2 | 0,04 | 0,14 | -0,02 | 0,23 | 0,30 | 1251 |
| NM_021805 | 0,02 | 0,05 | -0,11 | 0,29 | 0,21 | 1252 |
| NM_004590 | 0,04 | 0,26 | 0,10 | 0,27 | 0,27 | 1253 |
| AA482392 | 0,01 | -0,19 | -0,35 | 0,25 | 0,20 | 1254 |
| AA131826 | 0,01 | -0,04 | -0,20 | 0,26 | 0,17 | 1255 |
| AA947111 | 0,02 | 0,07 | -0,09 | 0,17 | 0,27 | 1256 |
| AI159796 | 0,04 | -0,13 | -0,28 | 0,20 | 0,28 | 1257 |
| AF086537 | 0,05 | 0,15 | -0,01 | 0,32 | 0,24 | 1258 |
| AI147932 | 0,00 | 0,13 | -0,03 | 0,23 | 0,16 | 1259 |
| AA460956 | 0,04 | 0,11 | -0,04 | 0,30 | 0,24 | 1260 |
| AA398249 | 0,03 | -0,11 | -0,27 | 0,23 | 0,26 | 1261 |
| H08161 | 0,04 | -0,23 | -0,39 | 0,23 | 0,28 | 1262 |
| AA281734 | 0,03 | -0,12 | -0,28 | 0,31 | 0,19 | 1263 |
| AA628488 | 0,04 | -0,18 | -0,33 | 0,20 | 0,30 | 1264 |
| AA430519 | 0,04 | -0,06 | -0,21 | 0,22 | 0,26 | 1265 |
| AA468113 | 0,05 | -0,16 | -0,31 | 0,29 | 0,25 | 1266 |
| AI424466 | 0,04 | 0,06 | -0,10 | 0,26 | 0,24 | 1267 |
| AI190760 | 0,04 | 0,04 | -0,11 | 0,29 | 0,23 | 1268 |
| N89992 | 0,01 | -0,06 | -0,21 | 0,23 | 0,18 | 1269 |
| AA046092 | 0,01 | 0,10 | -0,05 | 0,16 | 0,21 | 1270 |
| W35358 | 0,02 | 0,05 | -0,10 | 0,22 | 0,20 | 1271 |
| AA398341 | 0,04 | -0,19 | -0,33 | 0,27 | 0,23 | 1272 |
| H01969 | 0,05 | -0,09 | -0,24 | 0,32 | 0,20 | 1273 |
| AA970008 | 0,05 | -0,34 | -0,48 | 0,32 | 0,21 | 1274 |
| R89846 | 0,01 | 0,14 | -0,01 | 0,20 | 0,20 | 1275 |
| H18639 | 0,04 | 0,13 | -0,02 | 0,26 | 0,24 | 1276 |
| AI016342 | 0,02 | 0,02 | -0,12 | 0,18 | 0,22 | 1277 |
| NM_002184 | 0,04 | -0,23 | -0,37 | 0,17 | 0,28 | 1278 |
| NM_001643.1 | 0,03 | 0,13 | -0,01 | 0,19 | 0,26 | 1279 |
| AA280029 | 0,04 | -0,14 | -0,28 | 0,28 | 0,22 | 1280 |
| AA927949 | 0,00 | 0,17 | 0,02 | 0,16 | 0,14 | 1281 |
| AA625552 | 0,04 | 0,05 | -0,09 | 0,28 | 0,20 | 1282 |
| AA458912 | 0,03 | -0,23 | -0,37 | 0,24 | 0,23 | 1283 |
| AI188025 | 0,02 | 0,29 | 0,15 | 0,21 | 0,22 | 1284 |
| KM_007417 | 0,02 | 0,00 | -0,14 | 0,21 | 0,19 | 1285 |
| AA019529 | 0,03 | -0,29 | -0,42 | 0,22 | 0,22 | 1286 |
| AA401542 | 0,04 | -0,09 | -0,22 | 0,17 | 0,25 | 1287 |
| AI478746 | 0,04 | 0,00 | -0,13 | 0,23 | 0,22 | 1288 |
| AA291522 | 0,01 | -0,33 | -0,47 | 0,14 | 0,22 | 1289 |
| AI493122 | 0,05 | 0,16 | 0,03 | 0,25 | 0,22 | 1290 |
| AI203665 | 0,02 | 0,11 | -0,02 | 0,22 | 0,18 | 1291 |
| R74060 | 0,05 | -0,15 | -0,28 | 0,20 | 0,24 | 1292 |
| AI185721 | 0,04 | -0,25 | -0,37 | 0,22 | 0,19 | 1293 |
| AA437106 | 0,05 | 0,10 | -0,02 | 0,23 | 0,20 | 1294 |
| NM_139208 | 0,04 | -0,07 | -0,18 | 0,22 | 0,20 | 1295 |
| AI922221 | 0,05 | -0,02 | -0,14 | 0,20 | 0,20 | 1296 |
| AA412418 | 0,05 | -0,26 | -0,37 | 0,19 | 0,18 | 1297 |

Diese in Tabelle 2 und 3 charakteristischen Veränderungen sind für die erfindungsgemäße Verwendung gemäß Anspruch 1 ausnutzbar.

Die in den Tabellen 2 und 3 aufgeführten GenBank Accession Nummern (Internet-Zugang über http://www.ncbi.nlm.nih.gov/) der einzelnen Sequenzen sind in dem dieser Anmeldung angefügten Sequenzprotokol im Einzelnen jeweils einer Sequenz ID (Sequenz ID: 1 bis zur Sequenz ID: 1297) zugeordnet.

### Referenzen

1. Natanson C (1997) Anti-inflammatory therapies to treat sepsis and septic shock: A reassessment. Crit Care Med 25: 1095-1099
2. Geiger K (1995) Frühparameter für Multiorgandysfunktionssyndrom. in Hartenauer U (ed.) Sepsis in der Frühphase München MMV Medizin Verlag 19-25
3. Knaus WA , Draper EA, Wagner DP, Zimmermann JE (1985) Prognosis in acute organ-system failure. Ann Surg 202: 658-693
4. Goris RI, Bockhorst TP , Nuytinck JKS (1995) Mulitiple organ failure. Arch Surg 120:1109-1115
5. Vincent JL, Moreno R, Takala J, et al. (1996) The SOFA (Sepsis-related Organ Failure Assessment) score to describe organ dysfunction/failure. On behalf of the Working Group on Sepsis-Related Problems of the European Society of Intensive Care Medicine, Intensive Care Med. Jul 22(7):707-10.
6. Pfeiffer L, Ehrhardt N, Kretschmar R, et al. (1996) Endotoxinämie und Multiorganversagen nach Polytrauma. Anaesthesiol Reanimat 21: 91-96
7. Schlag G, Redl H (1993) Organ in shock, early organ failure, late organ failure., in Schlag G and Redl H (eds.) Pathophysiology of shock, sepsis, and organ failure Berlin Heidelberg Springer-Verlag, 1-4
8. Bone RC, Balk RA, Cerra FB, et al. (1992) The ACCP/SCCM Consensus Conference Committee (1992) Definitions for Sepsis and organ failure and guidelines for the use of innovative therapies in Sepsis. Chest 101:1656-1662; und Crit Care Med 1992; 20: 864-874.
9. Levy MM, Fink M, Marshall JC, et al. (2003) For the International Sepsis Definitions Conference: 2001 SCCM/ESICM/ACCP/ATS/SIS International Sepsis Definitions Conference. Crit Care Med. Apr;31 (4):1250-6
10.http://chirinn.klinikum.uni-muenchen.de/forschung/for_01_14_04.html, Stand Otober 2004, modifiziert
11.Marik PE. (1993) Gastric intramucosal pH. A better predictor of multiorgan dysfuction syndrom and death than oxygen derived variables in patients with sepsis. CHEST 104:, 225-229
12. Bernardin G , Pradier C, Tiger F, et al. (1996) Blood pressure and arterial lactate level are early indicators of short-term survival in human septic shock. Intensiv Care Med 22: 17-25;
13.Marecaux G, Pinsky MR, Dupont E, et al. (1996) Blood lactate levels are better prognostic indicators than TNF and IL-6 levels in patients with septic shock. Intensiv Care Med 22: 404-408
14.Duswald KH , Jochum M , Schramm W , Fritz H (1985) Released granulocytic elastase: an indicator of pathobiochemical alterations in septicemia after abdominal surgery. Surgery 98: 892-899
15. Nuytinck JKS, Goris RI, Redl H, et al. (1986) Posttraumatic complications and inflammatory mediators. Arch Surg 121: 886-890
16.Nast-Kolb D, Jochum M, Waydlas C, et al. (1991) Die Wertigkeit biochemischer Faktoren beim Polytrauma. Hefte Unfallheilkunde 215: 215
17. Hack CE, de Groot ER , Felt-Bersma RJ , et al. (1989): Increased plasma levels of interleukin-6 in sepsis" Blood 74: 1704-1710
18.Patel RT, Deen KI, Youngs D, et al. (1994) Interleukin 6 is a prognostic indicator of outcome in severe intra-abdominal sepsis. Br J Surg 81:1306-1308
19. Southern EM (1974) An improved method for transferring nucleotides from electrophoresis strips to thin layers of ion-exchange cellulose. Anal Biochem 62:317-318
20. Gillespie D, Spiegelman S (1965) A quantitative assay for DNA-RNA hybrids with DNA immobilized on a membrane. J Mol Biol 12:829-842
21. Lennon GG, Lehrach H (1991) Hybridization analyses of arrayed cDNA libraries. Trends Genet 7: 314-317
22. Kafatos FC, Jones CW, Efstratiadis A (1979) Determination of nucleic acid sequence homologies and relative concentrations by a dot hybridization procedure. Nucl Acid Res 7:1541-1552
23. Fodor SP, Read JL, Pirrung MC, Stryer L, Lu AT, Solas D (1991) Lightdirected, spatially addressable parallel chemical synthesis. Science 251:767-773
24. Pease AC, Solas D, Sullivan EJ, Cronin MT, Holmes CP, Fodor SP (1994) Light-generated oligonucleotide arrays for rapid DNA sequence analysis. Proc Natl Acad Sci USA 91:5022-5026
25. Schena M, Shalon D, Davis RW, Brown PO (1995) Quantitative monitoring of gene expression patterns with a complementary DNA microarray. Science 270:467-470
26. Golub TR, Slonim DK, Tamayo P, et al. (1999) Molecular classification of cancer: class discovery and class prediction by gene expression monitoring. Science 286:531-537
27.Alizadeh AA, Eisen MB, Davis RE, et al. (2000) Distinct types of diffuse large B-cell lymphoma identified by gene expression profiling. Nature 403:503-51
28. Feezor RJ, Baker HV, Xiao W, et al. (2004) Genomic and Proteomic Determinants of outcome in patients undergoing thoracoabdominal aortic aneurysm repair. Journal of Immunology 172 (11): 7103-7109
29. Huber W, Heydebreck A, Sueltmann H, et al. (2003) Parameter estimation for the calibration and variance stabilization of microarray data. Stat. Appl. in Gen. and Mol. Biol.. Vol. 2, Issue 1, Article 3
30. Prucha M et al. (2004) Expression profiling: Toward an application in sepsis diagnbstics. Shock, 22 (1): 29-33
31. Heller R A et al. (1997) Discovery and analysis of inflammatory diseaserelated genes using cDNA microarrays. PNAS 94: 2150-2155
32. Details for HG-U95AV2:2024_S_AT. (2004-10-1) https://www.affymetrix.com/analysis/ne taffx/fullrecord.affx?pk=H
33. Haslinger Christian et al. (2004) Microarray gene expression profiling of B-cell chronic lymphocytic leukemia subgroups defined by genomic aberrations and VH mutation status. JCO 19: 3937-3949

## Patentansprüche

1. Verwendung von in vitro aus einer Patientenprobe erhaltenen Genexpressionsprofilen für die Unterscheidung zwischen nichtinfektiösen und infektiösen Ursachen eines Multiorganversagens, wobei Genexpressionsprofile erhältlich sind aus einem Verfahren zur in vitro Messung von Genexpressionsprofilen, wobei
a. man in Patienten die Genexpression einer Mehrzahl von bestimmten, mit den nichtinfektiösen und infektiösen Ursachen eines Multiorganversagens in Zusammenhang stehenden Genen in einer Patientenprobe bestimmt,
b. die für die Unterscheidung von nichtinfektiösen und infektiösen Ursachen eines Multiorganversagens spezifischen Gene und/oder Genfragmente ausgewählt werden aus der Gruppe bestehend aus: SEQ-ID No. 1 bis SEQ-ID No. 1297 sowie Genfragmenten davon mit wenigstens 20-200 Nukleotiden, und
c. die aus der Gruppe bestehend aus: SEQ-ID No. 1 bis SEQ-ID No. 721 ausgewählten Gene und/oder Genfragmente bei Patienten mit infektiösem MOV signifikant gesteigerte Expressionswerte aufweisen im Vergleich zu den Patienten mit nichtinfektiösem MOV und/oder
die aus der Gruppe bestehend aus: SEQ-ID No. 722 bis SEQ-ID No. 1297 ausgewählten Gene und/oder Genfragmente bei Patienten mit infektiösem MOV signifikant reduzierte Expressionswerte aufweisen im Vergleich zu den Patienten mit nichtinfektiösem MOV.

2. Verwendung nach Anspruch 1, wobei die erhaltenen Genexpressionsdaten zur Herstellung von Software für die Beschreibung der individuellen Prognose eines Patienten, für Diagnosezwecke und/oder Patientendatenmangementsysteme, verwendet werden.

3. Verwendung nach Anspruch 1, wobei die Genexpressionsdaten zur Herstellung von klinischen Expertensystemen und/oder zur Modellierung von zelluläreren Signalübertragungswegen verwendet werden.

4. Verwendung nach einem der Ansprüche 1 bis 3, wobei die Genfragmente 20-80 Nukleotide umfassen.

5. Verwendung nach einem der Ansprüche 1 bis 4, **dadurch gekennzeichnet, dass** die Genexpressionsprofile mittels Hybridisierungsverfahren, insbesondere solchen auf Microarrays, ermittelt werden:

## Claims

1. Use of gene expression profiles obtained in vitro from patient samples for differentiating between the non-infectious and infectious causes of multiple organ failure, the gene expression profiles being obtainable from a method for in vitro measurement of gene expression profiles, wherein:
a. the patient's gene expression of a plurality of specific genes related to non-infectious and infectious causes of multiple organ failure is determined in a patient's sample, and
b. the genes and/or gene fragments specific for differentiating non-infectious and infectious causes of multiple organ failure are selected from a group consisting of: sequence ID No. 1 to sequence ID No. 1297 as well as gene fragments thereof with at least 20-200 nucleotides, and
c. the genes and/or gene fragments selected from the group consisting of sequence ID No.1 to 721 show significantly elevated expression values of patients with infectious MOF when compared to the patients with non-infectious MOF and/or
genes and/or gene fragments selected from the group consisting of sequence ID No. 722 to 1297 show significantly decreased expression values of patients with infectious MOF when compared to the patients with non-infectious MOF.

2. Use according to claim 1, wherein the obtained gene expression data are used for the production of software for the description of the individual prognosis of a patient, for diagnostic purposes and/or patient data management systems.

3. Use according to claim 1, wherein the gene activity data are used for the production of clinical expert systems and/or for modeling of cellular signal transmission paths.

4. Use according to one of claims 1 to 3, wherein the gene fragments comprise 20-80 nucleotides.

5. Use according to one of claims 1 to 4, **characterized in that** the gene expression profiles are determined by means of hybridizing methods, in particular those conducted on microarrays.

## Revendications

1. Utilisation de profils d'expression génique, obtenus in vitro à partir d'un échantillon de patients, pour faire une distinction entre des causes non infectieuses et des causes infectieuses d'une défaillance multi-organes, les profils d'expression génique pouvant être obtenus par un procédé de mesure in vitro de profils d'expression génique, pour laquelle :
a. on détermine, sur des patients, l'expression génique d'un grand nombre de certains gènes dans un échantillon de patients, associés aux causes non infectieuses et aux causes infectieuses d'une défaillance multi-organes,
b. on choisit les gènes et/ou les fragments géniques spécifiques de la distinction entre des causes non infectieuses et des causes infectieuses d'une défaillance multi-organes, dans le groupe consistant en SEQ ID NO:1 à SEQ ID N0:1297, ainsi que les fragments géniques qui en dérivent, ayant au moins 20-200 nucléotides, et
c. les gènes et/ou fragments géniques choisis dans le groupe consistant en SEQ ID NO:1 1 à SEQ ID NO:721 présentent, chez les patients présentant une MOV infectieuse, des valeurs de l'expression significativement plus élevées que les patients présentant une MOV non infectieuse, et/ou les gènes et/ou fragments géniques choisis dans le groupe consistant en SEQ ID NO:722 à SEQ ID NO:1297 présentent, chez les patients ayant une MOV infectieuse, des valeurs de l'expression significativement réduites par comparaison avec les patients présentant une MOV non infectieuse.

2. Utilisation selon la revendication 1, pour laquelle les données obtenues de l'expression génique sont utilisées pour la fabrication d'un logiciel pour la description du pronostic individuel d'un patient, à des fins diagnostiques, et/ou pour des systèmes de gestion des données patients.

3. Utilisation selon la revendication 1, pour laquelle les données de l'expression génique sont utilisées pour la fabrication de systèmes experts cliniques et/ou pour la modélisation de voies de transmission cellulaire de signaux.

4. Utilisation selon l'une des revendications 1 à 3, pour laquelle les fragments géniques comprennent 20 à 80 nucléotides.

5. Utilisation selon l'une des revendications 1 à 4, **caractérisée en ce que** les profils d'expression génique sont déterminés par des procédés d'hybridation, en particulier sur des micro-réseaux.
